# EUROPEAN PATENT SPECIFICATION

(11) **EP 0 910 652 B1**
(45) Date of publication and mention of the grant of the patent: **08.10.2014**
(21) Application number: 97917146.9
(22) Date of filing: 27.03.1997
(51) Int. Cl.: C12N 15/85, C12N 15/12, C12N 5/10, C07K 14/72, A01K 67/027

(54) **HORMONE-MEDIATED METHODS FOR MODULATING EXPRESSION OF EXOGENOUS GENES IN MAMMALIAN SYSTEMS, AND PRODUCTS RELATED THERETO**
HORMON-VERMITTELTE METHODEN ZUR BEEINFLUSSUNG DER EXPRESSION EXOGENER GENE IN SÄUGETIERSYSTEMEN UND DAMIT VERBUNDENE PRODUKTE
TECHNIQUES LIEES A UNE UTILISATION D'HORMONES VISANT A MODULER L'EXPRESSION DE GENES EXOGENES CHEZ DES MAMMIFERES ET PRODUITS CONNEXES

(30) Priority: 05.04.1996 US 628830
(43) Date of publication of application: 28.04.1999
(73) Proprietor: THE SALK INSTITUTE FOR BIOLOGICAL STUDIES, La Jolla California 92037 (US)
(72) Inventor: EVANS, Ronald, M., La Jolla, CA 92037 (US); NO, David, San Diego, CA 92109 (US)
(74) Representative: HOFFMANN EITLE
(86) International application number: PCT/US1997/005330
(87) International publication number: WO 1997/038117

(56) References cited:
- WO-A-91/13167
- WO-A-92/16546
- WO-A-93/03162
- WO-A-94/01558
- CELL, vol. 71, no. 1, 2 October 1992, pages 63-72, XP002036325 YAO T. ET AL.: "Drosophila ultraspiracle modulates ecdysone receptor function via heterodimer formation" cited in the application
- NATURE, vol. 362, 1 April 1993, pages 471-475, XP002036326 THOMAS H. ET AL.: "Heterodimerization of the Drosophila ecdysone receptor with retinoid X receptor and ultraspiracle"
- NATURE, vol. 366, 2 December 1993, pages 476-479, XP002036327 YAO T. ET AL.: "Functional ecdysone receptor is the product of EcR and ultraspiracle genes" cited in the application
- PNAS U.S.A., vol. 93, no. 8, 16 April 1996, pages 3346-3351, XP002036328 NO D. ET AL.: "Ecdysone-inducible gene expression in mammalian cells and transgenic mice"
- TAN NGUAN SOON ET AL: "MOLECULAR CLONING AND SEQUENCING OF THE HORMONE-BINDING DOMAIN OF OREOCHROMIS AUREUS ESTROGEN RECEPTOR GENE", DNA SEQUENCE, NEW YORK, NY, US, vol. 5, no. 6, 1 January 1995 (1995-01-01) , pages 359-370, XP009073331, ISSN: 1042-5179

### SEQUENCE LISTING

(1) GENERAL INFORMATION: (i) APPLICANT: THE SALK INSTITUTE FOR BIOLOGICAL STUDIES et al.
(ii) TITLE OF INVENTION: HORMONE-MEDIATED METHODS FOR MODULATING EXPRESSION OF EXOGENOUS GENES IN MAMMALIAN SYSTEMS, AND PRODUCTS RELATED THERETO
(iii) NUMBER OF SEQUENCES: 18
(iv) CORRESPONDENCE ADDRESS:
   (A) ADDRESSEE: Gray Cary Ware & Freidenrich
   (B) STREET: 4365 Executive Drive, Suite 1600
   (C) CITY: San Diego
   (D) STATE: CA
   (E) COUNTRY: USA
   (F) ZIP: 92121
(v) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25
(vi) CURRENT APPLICATION DATA:
   (A) APPLICATION NUMBER: PCT/US 97/05330
   (B) FILING DATE: 27/03/1997
   (C) CLASSIFICATION:
(viii) ATTORNEY/AGENT INFORMATION:
   (A) NAME: Reiter, Stephen E.
   (B) REGISTRATION NUMBER: 31,192
   (C) REFERENCE/DOCKET NUMBER: SALK1520WO
(ix) TELECOMMUNICATION INFORMATION:
   (A) TELEPHONE: 619-677-1409
   (B) TELEFAX: 619-677-1465
(2) INFORMATION FOR SEQ ID NO:1:
   (i) SEQUENCE CHARACTERISTICS:
      (A) LENGTH: 71 amino acids
      (B) TYPE: amino acid
      (C) STRANDEDNESS: single
      (D) TOPOLOGY: both
   (ii) MOLECULE TYPE: protein
   (v) FRAGMENT TYPE: internal
   (xi) SEQUENCE DESCRIPTION: SEQ ID NO:1:
   Cys Xaa Xaa Cys Xaa Xaa Asp Xaa Ala Xaa Gly Xaa Tyr Xaa Xaa Xaa
   1 5 10 15
   Xaa Cys Xaa Xaa Cys Lys Xaa Phe Phe Xaa Arg Xaa Xaa Xaa Xaa Xaa
   20 25 30
   Xaa Xaa Xaa Xaa Xaa Xaa Cys Xaa Xaa Xaa Xaa Xaa Xaa Xaa Xaa Cys
   35 40 45
   Xaa Xaa Xaa Lys Xaa Xaa Arg Xaa Xaa Cys Xaa Xaa Cys Arg Xaa Xaa
   50 55 60
   Lys Cys Xaa Xaa Xaa Gly Met
   65 70
   (2) INFORMATION FOR SEQ ID NO:2: (i) SEQUENCE CHARACTERISTICS: (A) LENGTH: 5 amino acids (B) TYPE: amino acid (C) STRANDEDNESS: single (D) TOPOLOGY: unknown (ii) MOLECULE TYPE: peptide (v) FRAGMENT TYPE: internal (xi) SEQUENCE DESCRIPTION: SEQ ID NO:2:
   (2) INFORMATION FOR SEQ ID NO:3: (i) SEQUENCE CHARACTERISTICS: (A) LENGTH: 5 amino acids (B) TYPE: amino acid (C) STRANDEDNESS: single (D) TOPOLOGY: unknown (ii) MOLECULE TYPE: peptide (v) FRAGMENT TYPE: internal (xi) SEQUENCE DESCRIPTION: SEQ ID NO:3:
(2) INFORMATION FOR SEQ ID NO:4:
   (i) SEQUENCE CHARACTERISTICS:
      (A) LENGTH: 2241 base pairs
      (B) TYPE: nucleic acid
      (C) STRANDEDNESS: both
      (D) TOPOLOGY: both
   (ii) MOLECULE TYPE: cDNA
   (ix) FEATURE:
      (A) NAME/KEY: CDS
      (B) LOCATION: 1..2241
      (D) OTHER INFORMATION: /product= "VgEcR"
   (xi) SEQUENCE DESCRIPTION: SEQ ID NO:4:
   ATG GCC CCC CCG ACC GAT GTC AGC CTG GGG GAC GAG CTC CAC TTA GAC 48
   Met Ala Pro Pro Thr Asp Val Ser Leu Gly Asp Glu Leu His Leu Asp
   1 5 10 15
   GGC GAG GAC GTG GCG ATG GCG CAT GCC GAC GCG CTA GAC GAT TTC GAT 96
   Gly Glu Asp Val Ala Met Ala His Ala Asp Ala Leu Asp Asp Phe Asp
   20 25 30
   CTG GAC ATG TTG GGG GAC GGG GAT TCC CCG GGT CCG GGA TTT ACC CCC 144
   Leu Asp Met Leu Gly Asp Gly Asp Ser Pro Gly Pro Gly Phe Thr Pro
   35 40 45
   CAC GAC TCC GCC CCC TAC GGC GCT CTG GAT ATG GCC GAC TTC GAG TTT 192
   His Asp Ser Ala Pro Tyr Gly Ala Leu Asp Met Ala Asp Phe Glu Phe
   50 55 60
   GAG CAG ATG TTT ACC GAT GCC CTT GGA ATT GAC GAG TAC GGT GGG AAG 240
   Glu Gln Met Phe Thr Asp Ala Leu Gly Ile Asp Glu Tyr Gly Gly Lys
   65 70 75 80
   CTT CTA GGT ACC TCT AGA AGG ATA TCG AAT TCT ATA TCT TCA GGT CGC 288
   Leu Leu Gly Thr Ser Arg Arg Ile Ser Asn Ser Ile Ser Ser Gly Arg
   85 90 95
   GAT GAT CTC TCG CCT TCG AGC AGC TTG AAC GGA TAC TCG GCG AAC GAA 336
   Asp Asp Leu Ser Pro Ser Ser Ser Leu Asn Gly Tyr Ser Ala Asn Glu
   100 105 110
   AGC TGC GAT GCG AAG AAG AGC AAG AAG GGA CCT GCG CCA CGG GTG CAA 384
   Ser Cys Asp Ala Lys Lys Ser Lys Lys Gly Pro Ala Pro Arg Val Gln
   115 120 125
   GAG GAG CTG TGC CTG GTT TGC GGC GAC AGG GCC TCC GGC TAC CAC TAC 432
   Glu Glu Leu Cys Leu Val Cys Gly Asp Arg Ala Ser Gly Tyr His Tyr
   130 135 140
   AAC GCC CTC ACC TGT GGA TCC TGC AAG GTG TTC TTT CGA CGC AGC GTT 480
   Asn Ala Leu Thr Cys Gly Ser Cys Lys Val Phe Phe Arg Arg Ser Val
   145 150 155 160
   ACG AAG AGC GCC GTC TAC TGC TGC AAG TTC GGG CGC GCC TGC GAA ATG 528
   Thr Lys Ser Ala Val Tyr Cys Cys Lys Phe Gly Arg Ala Cys Glu Met
   165 170 175
   GAC ATG TAC ATG AGG CGA AAG TGT CAG GAG TGC CGC CTG AAA AAG TGC 576
   Asp Met Tyr Met Arg Arg Lys Cys Gln Glu Cys Arg Leu Lys Lys Cys
   180 185 190
   CTG GCC GTG GGT ATG CGG CCG GAA TGC GTC GTC CCG GAG AAC CAA TGT
   624
   Leu Ala Val Gly Met Arg Pro Glu Cys Val Val Pro Glu Asn Gln Cys
   195 200 205
   GCG ATG AAG CGG CGC GAA AAG AAG GCC CAG AAG GAG AAG GAC AAA ATG
   672
   Ala Met Lys Arg Arg Glu Lys Lys Ala Gln Lys Glu Lys Asp Lys Met
   210 215 220
   ACC ACT TCG CCG AGC TCT CAG CAT GGC GGC AAT GGC AGC TTG GCC TCT
   720
   Thr Thr Ser Pro Ser Ser Gln His Gly Gly Asn Gly Ser Leu Ala Ser
   225 230 235 240
   GGT GGC GGC CAA GAC TTT GTT AAG AAG GAG ATT CTT GAC CTT ATG ACA
   768
   Gly Gly Gly Gln Asp Phe Val Lys Lys Glu Ile Leu Asp Leu Met Thr
   245 250 255
   TGC GAG CCG CCC CAG CAT GCC ACT ATT CCG CTA CTA CCT GAT GAA ATA
   816
   Cys Glu Pro Pro Gln His Ala Thr Ile Pro Leu Leu Pro Asp Glu Ile
   260 265 270
   TTG GCC AAG TGT CAA GCG CGC AAT ATA CCT TCC TTA ACG TAC AAT CAG
   864
   Leu Ala Lys Cys Gln Ala Arg Asn Ile Pro Ser Leu Thr Tyr Asn Gln
   275 280 285
   TTG GCC GTT ATA TAC AAG TTA ATT TGG TAC CAG GAT GGC TAT GAG CAG
   912
   Leu Ala Val Ile Tyr Lys Leu Ile Trp Tyr Gln Asp Gly Tyr Glu Gln
   290 295 300
   CCA TCT GAA GAG GAT CTC AGG CGT ATA ATG AGT CAA CCC GAT GAG AAC
   960
   Pro Ser Glu Glu Asp Leu Arg Arg Ile Met Ser Gln Pro Asp Glu Asn
   305 310 315 320
   GAG AGC CAA ACG GAC GTC AGC TTT CGG CAT ATA ACC GAG ATA ACC ATA
             1008
   Glu Ser Gln Thr Asp Val Ser Phe Arg His Ile Thr Glu Ile Thr Ile
                   325                 330                 335
   CTC ACG GTC CAG TTG ATT GTT GAG TTT GCT AAA GGT CTA CCA GCG TTT
             1056
   Leu Thr Val Gln Leu Ile Val Glu Phe Ala Lys Gly Leu Pro Ala Phe
               340                 345                 350
   ACA AAG ATA CCC CAG GAG GAC CAG ATC ACG TTA CTA AAG GCC TGC TCG
             1104
   Thr Lys Ile Pro Gln Glu Asp Gln Ile Thr Leu Leu Lys Ala Cys Ser
           355                 360                 365
   TCG GAG GTG ATG ATG CTG CGT ATG GCA CGA CGC TAT GAC CAC AGC TCG
             1152
   Ser Glu Val Met Met Leu Arg Met Ala Arg Arg Tyr Asp His Ser Ser
       370                 375                 380
   GAC TCA ATA TTC TTC GCG AAT AAT AGA TCA TAT ACG CGG GAT TCT TAC
             1200
   Asp Ser Ile Phe Phe Ala Asn Asn Arg Ser Tyr Thr Arg Asp Ser Tyr
   385                 390                 395                 400
   AAA ATG GCC GGA ATG GCT GAT AAC ATT GAA GAC CTG CTG CAT TTC TGC
             1248
   Lys Met Ala Gly Met Ala Asp Asn Ile Glu Asp Leu Leu His Phe Cys
                   405                 410                 415
   CGC CAA ATG TTC TCG ATG AAG GTG GAC AAC GTC GAA TAC GCG CTT CTC
             1296
   Arg Gln Met Phe Ser Met Lys Val Asp Asn Val Glu Tyr Ala Leu Leu
               420                 425                 430
   ACT GCC ATT GTG ATC TTC TCG GAC CGG CCG GGC CTG GAG AAG GCC CAA
             1344
   Thr Ala Ile Val Ile Phe Ser Asp Arg Pro Gly Leu Glu Lys Ala Gln
           435                 440                 445
   CTA GTC GAA GCG ATC CAG AGC TAC TAC ATC GAC ACG CTA CGC ATT TAT
   1392
   Leu Val Glu Ala Ile Gln Ser Tyr Tyr Ile Asp Thr Leu Arg Ile Tyr
   450 455 460
   ATA CTC AAC CGC CAC TGC GGC GAC TCA ATG AGC CTC GTC TTC TAC GCA
   1440
   Ile Leu Asn Arg His Cys Gly Asp Ser Met Ser Leu Val Phe Tyr Ala
   465 470 475 480
   AAG CTG CTC TCG ATC CTC ACC GAG CTG CGT ACG CTG GGC AAC CAG AAC
   1488
   Lys Leu Leu Ser Ile Leu Thr Glu Leu Arg Thr Leu Gly Asn Gln Asn
   485 490 495
   GCC GAG ATG TGT TTC TCA CTA AAG CTC AAA AAC CGC AAA CTG CCC AAG
   1536
   Ala Glu Met Cys Phe Ser Leu Lys Leu Lys Asn Arg Lys Leu Pro Lys
   500 505 510
   TTC CTC GAG GAG ATC TGG GAC GTT CAT GCC ATC CCG CCA TCG GTC CAG
   1584
   Phe Leu Glu Glu Ile Trp Asp Val His Ala Ile Pro Pro Ser Val Gln
   515 520 525
   TCG CAC CTT CAG ATT ACC CAG GAG GAG AAC GAG CGT CTC GAG CGG GCT
   1632
   Ser His Leu Gln Ile Thr Gln Glu Glu Asn Glu Arg Leu Glu Arg Ala
   530 535 540
   GAG CGT ATG CGG GCA TCG GTT GGG GGC GCC ATT ACC GCC GGC ATT GAT
   1680
   Glu Arg Met Arg Ala Ser Val Gly Gly Ala Ile Thr Ala Gly Ile Asp
   545 550 555 560
   TGC GAC TCT GCC TCC ACT TCG GCG GCG GCA GCC GCG GCC CAG CAT CAG
   1728
   Cys Asp Ser Ala Ser Thr Ser Ala Ala Ala Ala Ala Ala Gln His Gln
   565 570 575
   CCT CAG CCT CAG CCC CAG CCC CAA CCC TCC TCC CTG ACC CAG AAC GAT
   1776
   Pro Gln Pro Gln Pro Gln Pro Gln Pro Ser Ser Leu Thr Gln Asn Asp
   580 585 590
   TCC CAG CAC CAG ACA CAG CCG CAG CTA CAA CCT CAG CTA CCA CCT CAG
   1824
   Ser Gln His Gln Thr Gln Pro Gln Leu Gln Pro Gln Leu Pro Pro Gln
   595 600 605
   CTG CAA GGT CAA CTG CAA CCC CAG CTC CAA CCA CAG CTT CAG ACG CAA
   1872
   Leu Gln Gly Gln Leu Gln Pro Gln Leu Gln Pro Gln Leu Gln Thr Gln
   610 615 620
   CTC CAG CCA CAG ATT CAA CCA CAG CCA CAG CTC CTT CCC GTC TCC GCT
   1920
   Leu Gln Pro Gln Ile Gln Pro Gln Pro Gln Leu Leu Pro Val Ser Ala
   625 630 635 640
   CCC GTG CCC GCC TCC GTA ACC GCA CCT GGT TCC TTG TCC GCG GTC AGT
   1968
   Pro Val Pro Ala Ser Val Thr Ala Pro Gly Ser Leu Ser Ala Val Ser
   645 650 655
   ACG AGC AGC GAA TAC ATG GGC GGA AGT GCG GCC ATA GGA CCC ATC ACG
   2016
   Thr Ser Ser Glu Tyr Met Gly Gly Ser Ala Ala Ile Gly Pro Ile Thr
   660 665 670
   CCG GCA ACC ACC AGC AGT ATC ACG GCT GCC GTT ACC GCT AGC TCC ACC
   2064
   Pro Ala Thr Thr Ser Ser Ile Thr Ala Ala Val Thr Ala Ser Ser Thr
   675 680 685
   ACA TCA GCG GTA CCG ATG GGC AAC GGA GTT GGA GTC GGT GTT GGG GTG
   2112
   Thr Ser Ala Val Pro Met Gly Asn Gly Val Gly Val Gly Val Gly Val
   690 695 700
   GGC GGC AAC GTC AGC ATG TAT GCG AAC GCC CAG ACG GCG ATG GCC TTG
   2160
   Gly Gly Asn Val Ser Met Tyr Ala Asn Ala Gln Thr Ala Met Ala Leu
   705 710 715 720
   ATG GGT GTA GCC CTG CAT TCG CAC CAA GAG CAG CTT ATC GGG GGA GTG
   2208
   Met Gly Val Ala Leu His Ser His Gln Glu Gln Leu Ile Gly Gly Val
   725 730 735
   GCG GTT AAG TCG GAG CAC TCG ACG ACT GCA TAG
   2241
   Ala Val Lys Ser Glu His Ser Thr Thr Ala
   740 745
(2) INFORMATION FOR SEQ ID NO:5:
   (i) SEQUENCE CHARACTERISTICS:
      (A) LENGTH: 746 amino acids
      (B) TYPE: amino acid
      (D) TOPOLOGY: linear
   (ii) MOLECULE TYPE: protein
   (xi) SEQUENCE DESCRIPTION: SEQ ID NO:5:
   Met Ala Pro Pro Thr Asp Val Ser Leu Gly Asp Glu Leu His Leu Asp
   1 5 10 15
   Gly Glu Asp Val Ala Met Ala His Ala Asp Ala Leu Asp Asp Phe Asp
   20 25 30
   Leu Asp Met Leu Gly Asp Gly Asp Ser Pro Gly Pro Gly Phe Thr Pro
   35 40 45
   His Asp Ser Ala Pro Tyr Gly Ala Leu Asp Met Ala Asp Phe Glu Phe
   50 55 60
   Glu Gln Met Phe Thr Asp Ala Leu Gly Ile Asp Glu Tyr Gly Gly Lys
   65 70 75 80
   Leu Leu Gly Thr Ser Arg Arg Ile Ser Asn Ser Ile Ser Ser Gly Arg
   85 90 95
   Asp Asp Leu Ser Pro Ser Ser Ser Leu Asn Gly Tyr Ser Ala Asn Glu
   100 105 110
   Ser Cys Asp Ala Lys Lys Ser Lys Lys Gly Pro Ala Pro Arg Val Gln
   115 120 125
   Glu Glu Leu Cys Leu Val Cys Gly Asp Arg Ala Ser Gly Tyr His Tyr
   130 135 140
   Asn Ala Leu Thr Cys Gly Ser Cys Lys Val Phe Phe Arg Arg Ser Val
   145 150 155 160
   Thr Lys Ser Ala Val Tyr Cys Cys Lys Phe Gly Arg Ala Cys Glu Met
   165 170 175
   Asp Met Tyr Met Arg Arg Lys Cys Gln Glu Cys Arg Leu Lys Lys Cys
   180 185 190
   Leu Ala Val Gly Met Arg Pro Glu Cys Val Val Pro Glu Asn Gln Cys
   195 200 205
   Ala Met Lys Arg Arg Glu Lys Lys Ala Gln Lys Glu Lys Asp Lys Met
   210 215 220
   Thr Thr Ser Pro Ser Ser Gln His Gly Gly Asn Gly Ser Leu Ala Ser
   225 230 235 240
   Gly Gly Gly Gln Asp Phe Val Lys Lys Glu Ile Leu Asp Leu Met Thr
   245 250 255
   Cys Glu Pro Pro Gln His Ala Thr Ile Pro Leu Leu Pro Asp Glu Ile
   260 265 270
   Leu Ala Lys Cys Gln Ala Arg Asn Ile Pro Ser Leu Thr Tyr Asn Gln
   275 280 285
   Leu Ala Val Ile Tyr Lys Leu Ile Trp Tyr Gln Asp Gly Tyr Glu Gln
   290 295 300
   Pro Ser Glu Glu Asp Leu Arg Arg Ile Met Ser Gln Pro Asp Glu Asn
   305 310 315 320
   Glu Ser Gln Thr Asp Val Ser Phe Arg His Ile Thr Glu Ile Thr Ile
   325 330 335
   Leu Thr Val Gln Leu Ile Val Glu Phe Ala Lys Gly Leu Pro Ala Phe
   340 345 350
   Thr Lys Ile Pro Gln Glu Asp Gln Ile Thr Leu Leu Lys Ala Cys Ser
   355 360 365
   Ser Glu Val Met Met Leu Arg Met Ala Arg Arg Tyr Asp His Ser Ser
   370 375 380
   Asp Ser Ile Phe Phe Ala Asn Asn Arg Ser Tyr Thr Arg Asp Ser Tyr
   385 390 395 400
   Lys Met Ala Gly Met Ala Asp Asn Ile Glu Asp Leu Leu His Phe Cys
   405 410 415
   Arg Gln Met Phe Ser Met Lys Val Asp Asn Val Glu Tyr Ala Leu Leu
   420 425 430
   Thr Ala Ile Val Ile Phe Ser Asp Arg Pro Gly Leu Glu Lys Ala Gln
   435 440 445
   Leu Val Glu Ala Ile Gln Ser Tyr Tyr Ile Asp Thr Leu Arg Ile Tyr
   450 455 460
   Ile Leu Asn Arg His Cys Gly Asp Ser Met Ser Leu Val Phe Tyr Ala
   465 470 475 480
   Lys Leu Leu Ser Ile Leu Thr Glu Leu Arg Thr Leu Gly Asn Gln Asn
   485 490 495
   Ala Glu Met Cys Phe Ser Leu Lys Leu Lys Asn Arg Lys Leu Pro Lys
   500 505 510
   Phe Leu Glu Glu Ile Trp Asp Val His Ala Ile Pro Pro Ser Val Gln
   515 520 525
   Ser His Leu Gln Ile Thr Gln Glu Glu Asn Glu Arg Leu Glu Arg Ala
   530 535 540
   Glu Arg Met Arg Ala Ser Val Gly Gly Ala Ile Thr Ala Gly Ile Asp
   545 550 555 560
   Cys Asp Ser Ala Ser Thr Ser Ala Ala Ala Ala Ala Ala Gln His Gln
   565 570 575
   Pro Gln Pro Gln Pro Gln Pro Gln Pro Ser Ser Leu Thr Gln Asn Asp
   580 585 590
   Ser Gln His Gln Thr Gln Pro Gln Leu Gln Pro Gln Leu Pro Pro Gln
   595 600 605
   Leu Gln Gly Gln Leu Gln Pro Gln Leu Gln Pro Gln Leu Gln Thr Gln
   610 615 620
   Leu Gln Pro Gln Ile Gln Pro Gln Pro Gln Leu Leu Pro Val Ser Ala
   625 630 635 640
   Pro Val Pro Ala Ser Val Thr Ala Pro Gly Ser Leu Ser Ala Val Ser
   645 650 655
   Thr Ser Ser Glu Tyr Met Gly Gly Ser Ala Ala Ile Gly Pro Ile Thr
   660 665 670
   Pro Ala Thr Thr Ser Ser Ile Thr Ala Ala Val Thr Ala Ser Ser Thr
   675 680 685
   Thr Ser Ala Val Pro Met Gly Asn Gly Val Gly Val Gly Val Gly Val
   690 695 700
   Gly Gly Asn Val Ser Met Tyr Ala Asn Ala Gln Thr Ala Met Ala Leu
   705 710 715 720
   Met Gly Val Ala Leu His Ser His Gln Glu Gln Leu Ile Gly Gly Val
   725 730 735
   Ala Val Lys Ser Glu His Ser Thr Thr Ala
   740 745
(2) INFORMATION FOR SEQ ID NO:6:
   (i) SEQUENCE CHARACTERISTICS:
      (A) LENGTH: 2241 base pairs
      (B) TYPE: nucleic acid
      (C) STRANDEDNESS: both
      (D) TOPOLOGY: both
   (ii) MOLECULE TYPE: cDNA
   (ix) FEATURE:
      (A) NAME/KEY: CDS
      (B) LOCATION: 1..2241
      (D) OTHER INFORMATION: /product= "VpEcR"
   (xi) SEQUENCE DESCRIPTION: SEQ ID NO:6:
   ATG GCC CCC CCG ACC GAT GTC AGC CTG GGG GAC GAG CTC CAC TTA GAC
        48
   Met Ala Pro Pro Thr Asp Val Ser Leu Gly Asp Glu Leu His Leu Asp
    1               5                  10                  15
   GGC GAG GAC GTG GCG ATG GCG CAT GCC GAC GCG CTA GAC GAT TTC GAT
        96
   Gly Glu Asp Val Ala Met Ala His Ala Asp Ala Leu Asp Asp Phe Asp
                   20                  25                  30
   CTG GAC ATG TTG GGG GAC GGG GAT TCC CCG GGT CCG GGA TTT ACC CCC
       144
   Leu Asp Met Leu Gly Asp Gly Asp Ser Pro Gly Pro Gly Phe Thr Pro
               35                  40                  45
   CAC GAC TCC GCC CCC TAC GGC GCT CTG GAT ATG GCC GAC TTC GAG TTT
       192
   His Asp Ser Ala Pro Tyr Gly Ala Leu Asp Met Ala Asp Phe Glu Phe
           50                  55                  60
   GAG CAG ATG TTT ACC GAT GCC CTT GGA ATT GAC GAG TAC GGT GGG AAG
       240
   Glu Gln Met Phe Thr Asp Ala Leu Gly Ile Asp Glu Tyr Gly Gly Lys
    65                  70                  75                  80
   CTT CTA GGT ACC TCT AGA AGG ATA TCG AAT TCT ATA TCT TCA GGT CGC
       288
   Leu Leu Gly Thr Ser Arg Arg Ile Ser Asn Ser Ile Ser Ser Gly Arg
                    85                  90                  95
   GAT GAT CTC TCG CCT TCG AGC AGC TTG AAC GGA TAC TCG GCG AAC GAA
       336
   Asp Asp Leu Ser Pro Ser Ser Ser Leu Asn Gly Tyr Ser Ala Asn Glu
                100                 105                 110
   AGC TGC GAT GCG AAG AAG AGC AAG AAG GGA CCT GCG CCA CGG GTG CAA 384
   Ser Cys Asp Ala Lys Lys Ser Lys Lys Gly Pro Ala Pro Arg Val Gln
   115 120 125
   GAG GAG CTG TGC CTG GTT TGC GGC GAC AGG GCC TCC GGC TAC CAC TAC 432
   Glu Glu Leu Cys Leu Val Cys Gly Asp Arg Ala Ser Gly Tyr His Tyr
   130 135 140
   AAC GCC CTC ACC TGT GAG GGC TGC AAG GGG TTC TTT CGA CGC AGC GTT 480
   Asn Ala Leu Thr Cys Glu Gly Cys Lys Gly Phe Phe Arg Arg Ser Val
   145 150 155 160
   ACG AAG AGC GCC GTC TAC TGC TGC AAG TTC GGG CGC GCC TGC GAA ATG 528
   Thr Lys Ser Ala Val Tyr Cys Cys Lys Phe Gly Arg Ala Cys Glu Met
   165 170 175
   GAC ATG TAC ATG AGG CGA AAG TGT CAG GAG TGC CGC CTG AAA AAG TGC 576
   Asp Met Tyr Met Arg Arg Lys Cys Gln Glu Cys Arg Leu Lys Lys Cys
   180 185 190
   CTG GCC GTG GGT ATG CGG CCG GAA TGC GTC GTC CCG GAG AAC CAA TGT 624
   Leu Ala Val Gly Met Arg Pro Glu Cys Val Val Pro Glu Asn Gln Cys
   195 200 205
   GCG ATG AAG CGG CGC GAA AAG AAG GCC CAG AAG GAG AAG GAC AAA ATG 672
   Ala Met Lys Arg Arg Glu Lys Lys Ala Gln Lys Glu Lys Asp Lys Met
   210 215 220
   ACC ACT TCG CCG AGC TCT CAG CAT GGC GGC AAT GGC AGC TTG GCC TCT 720
   Thr Thr Ser Pro Ser Ser Gln His Gly Gly Asn Gly Ser Leu Ala Ser
   225 230 235 240
   GGT GGC GGC CAA GAC TTT GTT AAG AAG GAG ATT CTT GAC CTT ATG ACA
       768
   Gly Gly Gly Gln Asp Phe Val Lys Lys Glu Ile Leu Asp Leu Met Thr
                   245                 250                 255
   TGC GAG CCG CCC CAG CAT GCC ACT ATT CCG CTA CTA CCT GAT GAA ATA
       816
   Cys Glu Pro Pro Gln His Ala Thr Ile Pro Leu Leu Pro Asp Glu Ile
               260                 265                 270
   TTG GCC AAG TGT CAA GCG CGC AAT ATA CCT TCC TTA ACG TAC AAT CAG
       864
   Leu Ala Lys Cys Gln Ala Arg Asn Ile Pro Ser Leu Thr Tyr Asn Gln
           275                 280                 285
   TTG GCC GTT ATA TAC AAG TTA ATT TGG TAC CAG GAT GGC TAT GAG CAG
       912
   Leu Ala Val Ile Tyr Lys Leu Ile Trp Tyr Gln Asp Gly Tyr Glu Gln
       290                 295                 300
   CCA TCT GAA GAG GAT CTC AGG CGT ATA ATG AGT CAA CCC GAT GAG AAC
       960
   Pro Ser Glu Glu Asp Leu Arg Arg Ile Met Ser Gln Pro Asp Glu Asn
   305                 310                 315                 320
   GAG AGC CAA ACG GAC GTC AGC TTT CGG CAT ATA ACC GAG ATA ACC ATA
       1008
   Glu Ser Gln Thr Asp Val Ser Phe Arg His Ile Thr Glu Ile Thr Ile
                   325                 330                 335
   CTC ACG GTC CAG TTG ATT GTT GAG TTT GCT AAA GGT CTA CCA GCG TTT
       1056
   Leu Thr Val Gln Leu Ile Val Glu Phe Ala Lys Gly Leu Pro Ala Phe
               340                 345                 350
   ACA AAG ATA CCC CAG GAG GAC CAG ATC ACG TTA CTA AAG GCC TGC TCG
       1104
   Thr Lys Ile Pro Gln Glu Asp Gln Ile Thr Leu Leu Lys Ala Cys Ser
           355                 360                 365
   TCG GAG GTG ATG ATG CTG CGT ATG GCA CGA CGC TAT GAC CAC AGC TCG
       1152
   Ser Glu Val Met Met Leu Arg Met Ala Arg Arg Tyr Asp His Ser Ser
       370                 375                 380
   GAC TCA ATA TTC TTC GCG AAT AAT AGA TCA TAT ACG CGG GAT TCT TAC
       1200
   Asp Ser Ile Phe Phe Ala Asn Asn Arg Ser Tyr Thr Arg Asp Ser Tyr
   385                 390                 395                 400
   AAA ATG GCC GGA ATG GCT GAT AAC ATT GAA GAC CTG CTG CAT TTC TGC
       1248
   Lys Met Ala Gly Met Ala Asp Asn Ile Glu Asp Leu Leu His Phe Cys
                   405                 410                 415
   CGC CAA ATG TTC TCG ATG AAG GTG GAC AAC GTC GAA TAC GCG CTT CTC
       1296
   Arg Gln Met Phe Ser Met Lys Val Asp Asn Val Glu Tyr Ala Leu Leu
               420                 425                 430
   ACT GCC ATT GTG ATC TTC TCG GAC CGG CCG GGC CTG GAG AAG GCC CAA
       1344
   Thr Ala Ile Val Ile Phe Ser Asp Arg Pro Gly Leu Glu Lys Ala Gln
           435                 440                 445
   CTA GTC GAA GCG ATC CAG AGC TAC TAC ATC GAC ACG CTA CGC ATT TAT
       1392
   Leu Val Glu Ala Ile Gln Ser Tyr Tyr Ile Asp Thr Leu Arg Ile Tyr
       450                 455                 460
   ATA CTC AAC CGC CAC TGC GGC GAC TCA ATG AGC CTC GTC TTC TAC GCA
       1440
   Ile Leu Asn Arg His Cys Gly Asp Ser Met Ser Leu Val Phe Tyr Ala
   465                 470                 475                 480
   AAG CTG CTC TCG ATC CTC ACC GAG CTG CGT ACG CTG GGC AAC CAG AAC
       1488
   Lys Leu Leu Ser Ile Leu Thr Glu Leu Arg Thr Leu Gly Asn Gln Asn
                   485                 490                 495
   GCC GAG ATG TGT TTC TCA CTA AAG CTC AAA AAC CGC AAA CTG CCC AAG 1536
   Ala Glu Met Cys Phe Ser Leu Lys Leu Lys Asn Arg Lys Leu Pro Lys
   500 505 510
   TTC CTC GAG GAG ATC TGG GAC GTT CAT GCC ATC CCG CCA TCG GTC CAG 1584
   Phe Leu Glu Glu Ile Trp Asp Val His Ala Ile Pro Pro Ser Val Gln
   515 520 525
   TCG CAC CTT CAG ATT ACC CAG GAG GAG AAC GAG CGT CTC GAG CGG GCT 1632
   Ser His Leu Gln Ile Thr Gln Glu Glu Asn Glu Arg Leu Glu Arg Ala
   530 535 540
   GAG CGT ATG CGG GCA TCG GTT GGG GGC GCC ATT ACC GCC GGC ATT GAT 1680
   Glu Arg Met Arg Ala Ser Val Gly Gly Ala Ile Thr Ala Gly Ile Asp
   545 550 555 560
   TGC GAC TCT GCC TCC ACT TCG GCG GCG GCA GCC GCG GCC CAG CAT CAG 1728
   Cys Asp Ser Ala Ser Thr Ser Ala Ala Ala Ala Ala Ala Gln His Gln
   565 570 575
   CCT CAG CCT CAG CCC CAG CCC CAA CCC TCC TCC CTG ACC CAG AAC GAT 1776
   Pro Gln Pro Gln Pro Gln Pro Gln Pro Ser Ser Leu Thr Gln Asn Asp
   580 585 590
   TCC CAG CAC CAG ACA CAG CCG CAG CTA CAA CCT CAG CTA CCA CCT CAG 1824
   Ser Gln His Gln Thr Gln Pro Gln Leu Gln Pro Gln Leu Pro Pro Gln
   595 600 605
   CTG CAA GGT CAA CTG CAA CCC CAG CTC CAA CCA CAG CTT CAG ACG CAA 1872
   Leu Gln Gly Gln Leu Gln Pro Gln Leu Gln Pro Gln Leu Gln Thr Gln
   610 615 620
   CTC CAG CCA CAG ATT CAA CCA CAG CCA CAG CTC CTT CCC GTC TCC GCT
   1920
   Leu Gln Pro Gln Ile Gln Pro Gln Pro Gln Leu Leu Pro Val Ser Ala
   625 630 635 640
   CCC GTG CCC GCC TCC GTA ACC GCA CCT GGT TCC TTG TCC GCG GTC AGT
   1968
   Pro Val Pro Ala Ser Val Thr Ala Pro Gly Ser Leu Ser Ala Val Ser
   645 650 655
   ACG AGC AGC GAA TAC ATG GGC GGA AGT GCG GCC ATA GGA CCC ATC ACG
   2016
   Thr Ser Ser Glu Tyr Met Gly Gly Ser Ala Ala Ile Gly Pro Ile Thr
   660 665 670
   CCG GCA ACC ACC AGC AGT ATC ACG GCT GCC GTT ACC GCT AGC TCC ACC
   2064
   Pro Ala Thr Thr Ser Ser Ile Thr Ala Ala Val Thr Ala Ser Ser Thr
   675 680 685
   ACA TCA GCG GTA CCG ATG GGC AAC GGA GTT GGA GTC GGT GTT GGG GTG
   2112
   Thr Ser Ala Val Pro Met Gly Asn Gly Val Gly Val Gly Val Gly Val
   690 695 700
   GGC GGC AAC GTC AGC ATG TAT GCG AAC GCC CAG ACG GCG ATG GCC TTG
   2160
   Gly Gly Asn Val Ser Met Tyr Ala Asn Ala Gln Thr Ala Met Ala Leu
   705 710 715 720
   ATG GGT GTA GCC CTG CAT TCG CAC CAA GAG CAG CTT ATC GGG GGA GTG
   2208
   Met Gly Val Ala Leu His Ser His Gln Glu Gln Leu Ile Gly Gly Val
   725 730 735
   GCG GTT AAG TCG GAG CAC TCG ACG ACT GCA TAG
   2241
   Ala Val Lys Ser Glu His Ser Thr Thr Ala
   740 745
(2) INFORMATION FOR SEQ ID NO:7:
   (i) SEQUENCE CHARACTERISTICS:
      (A) LENGTH: 746 amino acids
      (B) TYPE: amino acid
      (D) TOPOLOGY: linear
   (ii) MOLECULE TYPE: protein

   (xi) SEQUENCE DESCRIPTION: SEQ ID NO:7:
   Met Ala Pro Pro Thr Asp Val Ser Leu Gly Asp Glu Leu His Leu Asp
   1 5 10 15
   Gly Glu Asp Val Ala Met Ala His Ala Asp Ala Leu Asp Asp Phe Asp
   20 25 30
   Leu Asp Met Leu Gly Asp Gly Asp Ser Pro Gly Pro Gly Phe Thr Pro
   35 40 45
   His Asp Ser Ala Pro Tyr Gly Ala Leu Asp Met Ala Asp Phe Glu Phe
   50 55 60
   Glu Gln Met Phe Thr Asp Ala Leu Gly Ile Asp Glu Tyr Gly Gly Lys
   65 70 75 80
   Leu Leu Gly Thr Ser Arg Arg Ile Ser Asn Ser Ile Ser Ser Gly Arg
   85 90 95
   Asp Asp Leu Ser Pro Ser Ser Ser Leu Asn Gly Tyr Ser Ala Asn Glu
   100 105 110
   Ser Cys Asp Ala Lys Lys Ser Lys Lys Gly Pro Ala Pro Arg Val Gln
   115 120 125
   Glu Glu Leu Cys Leu Val Cys Gly Asp Arg Ala Ser Gly Tyr His Tyr
   130 135 140
   Asn Ala Leu Thr Cys Glu Gly Cys Lys Gly Phe Phe Arg Arg Ser Val
   145 150 155 160
   Thr Lys Ser Ala Val Tyr Cys Cys Lys Phe Gly Arg Ala Cys Glu Met
   165 170 175
   Asp Met Tyr Met Arg Arg Lys Cys Gln Glu Cys Arg Leu Lys Lys Cys
   180 185 190
   Leu Ala Val Gly Met Arg Pro Glu Cys Val Val Pro Glu Asn Gln Cys
           195                 200                 205
   Ala Met Lys Arg Arg Glu Lys Lys Ala Gln Lys Glu Lys Asp Lys Met
       210                 215                 220
   Thr Thr Ser Pro Ser Ser Gln His Gly Gly Asn Gly Ser Leu Ala Ser
   225                 230                 235                 240
   Gly Gly Gly Gln Asp Phe Val Lys Lys Glu Ile Leu Asp Leu Met Thr
                   245                 250                 255
   Cys Glu Pro Pro Gln His Ala Thr Ile Pro Leu Leu Pro Asp Glu Ile
               260                 265                 270
   Leu Ala Lys Cys Gln Ala Arg Asn Ile Pro Ser Leu Thr Tyr Asn Gln
           275                 280                 285
   Leu Ala Val Ile Tyr Lys Leu Ile Trp Tyr Gln Asp Gly Tyr Glu Gln
       290                 295                 300
   Pro Ser Glu Glu Asp Leu Arg Arg Ile Met Ser Gln Pro Asp Glu Asn
   305                 310                 315                 320
   Glu Ser Gln Thr Asp Val Ser Phe Arg His Ile Thr Glu Ile Thr Ile
                   325                 330                 335
   Leu Thr Val Gln Leu Ile Val Glu Phe Ala Lys Gly Leu Pro Ala Phe
               340                 345                 350
   Thr Lys Ile Pro Gln Glu Asp Gln Ile Thr Leu Leu Lys Ala Cys Ser
           355                 360                 365
   Ser Glu Val Met Met Leu Arg Met Ala Arg Arg Tyr Asp His Ser Ser
       370                 375                 380
   Asp Ser Ile Phe Phe Ala Asn Asn Arg Ser Tyr Thr Arg Asp Ser Tyr
   385                 390                 395                 400
   Lys Met Ala Gly Met Ala Asp Asn Ile Glu Asp Leu Leu His Phe Cys
                   405                 410                 415
   Arg Gln Met Phe Ser Met Lys Val Asp Asn Val Glu Tyr Ala Leu Leu
               420                 425                 430
   Thr Ala Ile Val Ile Phe Ser Asp Arg Pro Gly Leu Glu Lys Ala Gln
   435 440 445
   Leu Val Glu Ala Ile Gln Ser Tyr Tyr Ile Asp Thr Leu Arg Ile Tyr
   450 455 460
   Ile Leu Asn Arg His Cys Gly Asp Ser Met Ser Leu Val Phe Tyr Ala
   465 470 475 480
   Lys Leu Leu Ser Ile Leu Thr Glu Leu Arg Thr Leu Gly Asn Gln Asn
   485 490 495
   Ala Glu Met Cys Phe Ser Leu Lys Leu Lys Asn Arg Lys Leu Pro Lys
   500 505 510
   Phe Leu Glu Glu Ile Trp Asp Val His Ala Ile Pro Pro Ser Val Gln
   515 520 525
   Ser His Leu Gln Ile Thr Gln Glu Glu Asn Glu Arg Leu Glu Arg Ala
   530 535 540
   Glu Arg Met Arg Ala Ser Val Gly Gly Ala Ile Thr Ala Gly Ile Asp
   545 550 555 560
   Cys Asp Ser Ala Ser Thr Ser Ala Ala Ala Ala Ala Ala Gln His Gln
   565 570 575
   Pro Gln Pro Gln Pro Gln Pro Gln Pro Ser Ser Leu Thr Gln Asn Asp
   580 585 590
   Ser Gln His Gln Thr Gln Pro Gln Leu Gln Pro Gln Leu Pro Pro Gln
   595 600 605
   Leu Gln Gly Gln Leu Gln Pro Gln Leu Gln Pro Gln Leu Gln Thr Gln
   610 615 620
   Leu Gln Pro Gln Ile Gln Pro Gln Pro Gln Leu Leu Pro Val Ser Ala
   625 630 635 640
   Pro Val Pro Ala Ser Val Thr Ala Pro Gly Ser Leu Ser Ala Val Ser
   645 650 655
   Thr Ser Ser Glu Tyr Met Gly Gly Ser Ala Ala Ile Gly Pro Ile Thr
   660 665 670
   Pro Ala Thr Thr Ser Ser Ile Thr Ala Ala Val Thr Ala Ser Ser Thr
   675 680 685
   Thr Ser Ala Val Pro Met Gly Asn Gly Val Gly Val Gly Val Gly Val
   690 695 700
   Gly Gly Asn Val Ser Met Tyr Ala Asn Ala Gln Thr Ala Met Ala Leu
   705 710 715 720
   Met Gly Val Ala Leu His Ser His Gln Glu Gln Leu Ile Gly Gly Val
   725 730 735
   Ala Val Lys Ser Glu His Ser Thr Thr Ala
   740 745
(2) INFORMATION FOR SEQ ID NO:8:
   (i) SEQUENCE CHARACTERISTICS:
      (A) LENGTH: 3126 base pairs
      (B) TYPE: nucleic acid
      (C) STRANDEDNESS: both
      (D) TOPOLOGY: both
   (ii) MOLECULE TYPE: cDNA
   (ix) FEATURE:
      (A) NAME/KEY: CDS
      (B) LOCATION: 1..3126
      (D) OTHER INFORMATION: /product= "GEcR"
   (xi) SEQUENCE DESCRIPTION: SEQ ID NO:8:
   ATG GAC TCC AAA GAA TCA TTA ACT CCT GGT AGA GAA GAA AAC CCC AGC
   48
   AGT GTG CTT GCT CAG GAG AGG GGA GAT GTG ATG GAC TTC TAT AAA ACC
   96
   CTA AGA GGA GGA GCT ACT GTG AAG GTT TCT GCG TCT TCA CCC TCA CTG
   144
   GCT GTC GCT TCT CAA TCA GAC TCC AAG CAG CGA AGA CTT TTG GTT GAT
   192
   TTT CCA AAA GGC TCA GTA AGC AAT GCG CAG CAG CCA GAT CTG TCC AAA
   240
   GCA GTT TCA CTC TCA ATG GGA CTG TAT ATG GGA GAG ACA GAA ACA AAA
   288
   GTG ATG GGA AAT GAC CTG GGA TTC CCA CAG CAG GGC CAA ATC AGC CTT
   336
   TCC TCG GGG GAA ACA GAC TTA AAG CTT TTG GAA GAA AGC ATT GCA AAC
   384
   CTC AAT AGG TCG ACC AGT GTT CCA GAG AAC CCC AAG AGT TCA GCA TCC
   432
   ACT GCT GTG TCT GCT GCC CCC ACA GAG AAG GAG TTT CCA AAA ACT CAC
   480
   TCT GAT GTA TCT TCA GAA CAG CAA CAT TTG AAG GGC CAG ACT GGC ACC
   528
   AAC GGT GGC AAT GTG AAA TTG TAT ACC ACA GAC CAA AGC ACC TTT GAC
   576
   ATT TTG CAG GAT TTG GAG TTT TCT TCT GGG TCC CCA GGT AAA GAG ACG
   624
   AAT GAG AGT CCT TGG AGA TCA GAC CTG TTG ATA GAT GAA AAC TGT TTG
   672
   CTT TCT CCT CTG GCG GGA GAA GAC GAT TCA TTC CTT TTG GAA GGA AAC
   720
   TCG AAT GAG GAC TGC AAG CCT CTC ATT TTA CCG GAC ACT AAA CCC AAA
   768
   ATT AAG GAT AAT GGA GAT CTG GTT TTG TCA AGC CCC AGT AAT GTA ACA
   816
   CTG CCC CAA GTG AAA ACA GAA AAA GAA GAT TTC ATC GAA CTC TGC ACC
   864
   CCT GGG GTA ATT AAG CAA GAG AAA CTG GGC ACA GTT TAC TGT CAG GCA 912
   AGC TTT CCT GGA GCA AAT ATA ATT GGT AAT AAA ATG TCT GCC ATT TCT 960
   GTT CAT GGT GTG AGT ACC TCT GGA GGA CAG ATG TAC CAC TAT GAC ATG 1008
   AAT ACA GCA TCC CTT TCT CAA CAG CAG GAT CAG AAG CCT ATT TTT AAT 1056
   GTC ATT CCA CCA ATT CCC GTT GGT TCC GAA AAT TGG AAT AGG TGC CAA 1104
   GGA TCT GGA GAT GAC AAC TTG ACT TCT CTG GGG ACT CTG AAC TTC CCT 1152
   GGT CGA ACA GTT TTT TCT AAT GGC TAT TCA AGC CCC AGC ATG AGA CCA 1200
   GAT GTA AGC TCT CCT CCA TCC AGC TCC TCA ACA GCA ACA ACA GGA CCA 1248
   CCT CCC AGC GGC CGC GTG CAA GAG GAG CTG TGC CTG GTT TGC GGC GAC 1296
   AGG GCC TCC GGC TAC CAC TAC AAC GCC CTC ACC TGT GGA TCC TGC AAG 1344
   GTG TTC TTT CGA CGC AGC GTT ACG AAG AGC GCC GTC TAC TGC TGC AAG 1392
   TTC GGG CGC GCC TGC GAA ATG GAC ATG TAC ATG AGG CGA AAG TGT CAG 1440
   GAG TGC CGC CTG AAA AAG TGC CTG GCC GTG GGT ATG CGG CCG GAA TGC 1488
   GTC GTC CCG GAG AAC CAA TGT GCG ATG AAG CGG CGC GAA AAG AAG GCC 1536
   CAG AAG GAG AAG GAC AAA ATG ACC ACT TCG CCG AGC TCT CAG CAT GGC 1584
   GGC AAT GGC AGC TTG GCC TCT GGT GGC GGC CAA GAC TTT GTT AAG AAG 1632
   GAG ATT CTT GAC CTT ATG ACA TGC GAG CCG CCC CAG CAT GCC ACT ATT
       1680
   CCG CTA CTA CCT GAT GAA ATA TTG GCC AAG TGT CAA GCG CGC AAT ATA
       1728
   CCT TCC TTA ACG TAC AAT CAG TTG GCC GTT ATA TAC AAG TTA ATT TGG
       1776
   TAC CAG GAT GGC TAT GAG CAG CCA TCT GAA GAG GAT CTC AGG CGT ATA
       1824
   ATG AGT CAA CCC GAT GAG AAC GAG AGC CAA ACG GAC GTC AGC TTT CGG
       1872
   CAT ATA ACC GAG ATA ACC ATA CTC ACG GTC CAG TTG ATT GTT GAG TTT
       1920
   GCT AAA GGT CTA CCA GCG TTT ACA AAG ATA CCC CAG GAG GAC CAG ATC
       1968
   ACG TTA CTA AAG GCC TGC TCG TCG GAG GTG ATG ATG CTG CGT ATG GCA
       2016
   CGA CGC TAT GAC CAC AGC TCG GAC TCA ATA TTC TTC GCG AAT AAT AGA 2064
   TCA TAT ACG CGG GAT TCT TAC AAA ATG GCC GGA ATG GCT GAT AAC ATT 2112
   GAA GAC CTG CTG CAT TTC TGC CGC CAA ATG TTC TCG ATG AAG GTG GAC 2160
   AAC GTC GAA TAC GCG CTT CTC ACT GCC ATT GTG ATC TTC TCG GAC CGG 2208
   CCG GGC CTG GAG AAG GCC CAA CTA GTC GAA GCG ATC CAG AGC TAC TAC 2256
   ATC GAC ACG CTA CGC ATT TAT ATA CTC AAC CGC CAC TGC GGC GAC TCA 2304
   ATG AGC CTC GTC TTC TAC GCA AAG CTG CTC TCG ATC CTC ACC GAG CTG 2352
   CGT ACG CTG GGC AAC CAG AAC GCC GAG ATG TGT TTC TCA CTA AAG CTC 2400
   AAA AAC CGC AAA CTG CCC AAG TTC CTC GAG GAG ATC TGG GAC GTT CAT 2448
   GCC ATC CCG CCA TCG GTC CAG TCG CAC CTT CAG ATT ACC CAG GAG GAG 2496
   AAC GAG CGT CTC GAG CGG GCT GAG CGT ATG CGG GCA TCG GTT GGG GGC 2544
   GCC ATT ACC GCC GGC ATT GAT TGC GAC TCT GCC TCC ACT TCG GCG GCG 2592
   GCA GCC GCG GCC CAG CAT CAG CCT CAG CCT CAG CCC CAG CCC CAA CCC 2640
   TCC TCC CTG ACC CAG AAC GAT TCC CAG CAC CAG ACA CAG CCG CAG CTA 2688
   CAA CCT CAG CTA CCA CCT CAG CTG CAA GGT CAA CTG CAA CCC CAG CTC 2736
   CAA CCA CAG CTT CAG ACG CAA CTC CAG CCA CAG ATT CAA CCA CAG CCA 2784
   CAG CTC CTT CCC GTC TCC GCT CCC GTG CCC GCC TCC GTA ACC GCA CCT
       2832
   GGT TCC TTG TCC GCG GTC AGT ACG AGC AGC GAA TAC ATG GGC GGA AGT
       2880
   GCG GCC ATA GGA CCC ATC ACG CCG GCA ACC ACC AGC AGT ATC ACG GCT
       2928
   GCC GTT ACC GCT AGC TCC ACC ACA TCA GCG GTA CCG ATG GGC AAC GGA
       2976
   GTT GGA GTC GGT GTT GGG GTG GGC GGC AAC GTC AGC ATG TAT GCG AAC
       3024
   GCC CAG ACG GCG ATG GCC TTG ATG GGT GTA GCC CTG CAT TCG CAC CAA
       3072
   GAG CAG CTT ATC GGG GGA GTG GCG GTT AAG TCG GAG CAC TCG ACG ACT
       3120
   GCA TAG
        3126
(2) INFORMATION FOR SEQ ID NO:9:
   (i) SEQUENCE CHARACTERISTICS:
      (A) LENGTH: 1041 amino acids (B) TYPE: amino acid (D) TOPOLOGY: linear
   (ii) MOLECULE TYPE: protein
   (xi) SEQUENCE DESCRIPTION: SEQ ID NO:9:
(2) INFORMATION FOR SEQ ID NO:10:
   (i) SEQUENCE CHARACTERISTICS:
      (A) LENGTH: 13 base pairs (B) TYPE: nucleic acid (C) STRANDEDNESS: both (D) TOPOLOGY: both
   (ii) MOLECULE TYPE: DNA (genomic)
   (ix) FEATURE:
      (A) NAME/KEY: misc_feature
      (B) LOCATION: 7
      (D) OTHER INFORMATION: /product= "Modified Ecdysone Response Element" nucleotides, /note= "N at position 7 is 0 up to 5 nucleotides, with 1 nucleotide being especially preferred."
   (xi) SEQUENCE DESCRIPTION: SEQ ID NO:10:
   RGBNNMNTGN NCY
   13
(2) INFORMATION FOR SEQ ID NO:11:
   (i) SEQUENCE CHARACTERISTICS:
      (A) LENGTH: 13 base pairs
      (B) TYPE: nucleic acid
      (C) STRANDEDNESS: both
      (D) TOPOLOGY: both
   (ii) MOLECULE TYPE: DNA (genomic)
   (ix) FEATURE:
      (A) NAME/KEY: misc_feature
      (B) LOCATION: 7
      (D) OTHER INFORMATION: /product= "Modified Ecdysone Response Element" /note= "N at position 7 can be 0 up to 5 nucleotides, with 1 nucleotide being preferred."
   (xi) SEQUENCE DESCRIPTION: SEQ ID NO:11:
   RGNNCANKNN VCY
   13
(2) INFORMATION FOR SEQ ID NO:12:
   (i) SEQUENCE CHARACTERISTICS:
      (A) LENGTH: 13 base pairs
      (B) TYPE: nucleic acid
      (C) STRANDEDNESS: both
      (D) TOPOLOGY: both
   (ii) MOLECULE TYPE: DNA (genomic)
   (xi) SEQUENCE DESCRIPTION: SEQ ID NO:12:
   AGTGCANTGT TCT
   13
(2) INFORMATION FOR SEQ ID NO:13:
   (i) SEQUENCE CHARACTERISTICS:
      (A) LENGTH: 13 base pairs
      (B) TYPE: nucleic acid
      (C) STRANDEDNESS: both
      (D) TOPOLOGY: both
   (ii) MOLECULE TYPE: DNA (genomic)
   (ix) FEATURE:
      (A) NAME/KEY: misc_feature
      (B) LOCATION: 7
      (D) OTHER INFORMATION: /product= "Ecdysone Response Element" /note= "N at position 7 can be 0 up to 5 preferred." nucleotides, with 3 nucleotides being
   (xi) SEQUENCE DESCRIPTION: SEQ ID NO:13:
   RGBNNMNRGB NNM 13
(2) INFORMATION FOR SEQ ID NO:14:
   (i) SEQUENCE CHARACTERISTICS:
      (A) LENGTH: 49 base pairs
      (B) TYPE: nucleic acid
      (C) STRANDEDNESS: both
      (D) TOPOLOGY: both
   (ii) MOLECULE TYPE: DNA (genomic)
   (xi) SEQUENCE DESCRIPTION: SEQ ID NO:14:
   TACAACGCCC TCACCTGTGG ATCCTGCAAG GTGTTTCTTT CGACGCAGC 49
(2) INFORMATION FOR SEQ ID NO:15:
   (i) SEQUENCE CHARACTERISTICS:
      (A) LENGTH: 53 base pairs (B) TYPE: nucleic acid (C) STRANDEDNESS: both (D) TOPOLOGY: both
   (ii) MOLECULE TYPE: DNA (genomic)
   (xi) SEQUENCE DESCRIPTION: SEQ ID NO:15:
   GTACTCCCGG GGCGGGGCTA TGCGGGGCGG GGCTAATCGC TAGGGGCGGG GCA 53
(2) INFORMATION FOR SEQ ID NO:16:
   (i) SEQUENCE CHARACTERISTICS:
      (A) LENGTH: 53 base pairs
      (B) TYPE: nucleic acid
      (C) STRANDEDNESS: both
      (D) TOPOLOGY: both
   (ii) MOLECULE TYPE: DNA (genomic)
   (xi) SEQUENCE DESCRIPTION: SEQ ID NO:16:
   GTACTGCCCC GCCCCTAGCG ATTAGCCCCG CCCCGCATAG CCCCGCCCCG GGA 53
(2) INFORMATION FOR SEQ ID NO:17:
   (i) SEQUENCE CHARACTERISTICS:
      (A) LENGTH: 34 base pairs
      (B) TYPE: nucleic acid
      (C) STRANDEDNESS: both
      (D) TOPOLOGY: both
   (ii) MOLECULE TYPE: DNA (genomic)
   (xi) SEQUENCE DESCRIPTION: SEQ ID NO:17:
   AGCTCGATGG ACAAGTGCAT TGTTCTTTGC TGAA
   34
(2) INFORMATION FOR SEQ ID NO:18:
   (i) SEQUENCE CHARACTERISTICS:
      (A) LENGTH: 35 base pairs
      (B) TYPE: nucleic acid
      (C) STRANDEDNESS: both
      (D) TOPOLOGY: both
   (ii) MOLECULE TYPE: DNA (genomic)
   (xi) SEQUENCE DESCRIPTION: SEQ ID NO:18:
   AGCTTTCAGC AAGAGAACAA TGCACTTGTC CATCG
   35

## Description

### FIELD OF THE INVENTION

The present invention relates to methods in the field of recombinant DNA technology, and products related thereto. More particularly, the invention relates to methods and products for modulating the expression of exogenous genes in mammalian systems.

### BACKGROUND OF THE INVENTION

The steroid/thyroid hormone receptors comprise a superfamily of ligand-dependent transcription factors that play a crucial role in mediating changes in cell fate and function (Evans, R.M., Science 240:889-895 (1988)). The receptors transduce extracellular hormonal signals to target genes that contain specific enhancer sequences referred to as hormone response elements (HREs) Evans, (1988); Green and Chambon, Trends Genet. 4:309-314 (1988); Yamamoto, K.R., Annu. Rev. Genet. 19:209-252 (1985)). Each receptor recognizes its own HRE, assuring that a distinct response is triggered by each hormonal signal. Together the collection of related transcription factors and their cognate response elements provides a unique opportunity to control gene expression.

The DNA binding domain of each member of the steroid/thyroid superfamily of receptors has 66-68 amino acids. Twenty of these, including nine cysteines, are conserved throughout the family. The modular structure of members of this receptor superfamily allows the exchange of homologous domains between receptors to create functional chimeras. This strategy was used to demonstrate that the DNA binding domain is solely responsible for the specific recognition of the HRE in vivo (Green and Chambon, Nature 325:75-78 (1987); Giguere et al., Nature 330:624-629 (1987); Petkovich et al., Nature 330:444-450 (1987); Kumar et al., Cell 51:941-951 (1987); Umesono et al., Nature 336:262-265 (1988); Thompson and Evans, Proc. Natl. Acad. Sci. U.S.A. 86:3494-3498 (1989) and in vitro (Kumar and Chambon, Cell 55:145-156 (1988)). By analogy with the proposed structure for Xenopus transcription factor IIIA (Miller et al., EMBO J. 4:1609-1614 (1985)), the invariant cysteines are thought to form two "zinc fingers" that mediate the DNA binding function (Hollenberg and Evans, Cell 55:899-906 (1988)). Involvement of these cysteines in Zn(II) coordination is supported by extended X-ray absorption fine structure (Freedman et al., Nature 334:543-546 (1988)), and DNA binding by point mutagenesis experiments (Hollenberg and Evans, (1988)); Severne et al., EMBO J. 7:2503-2508 (1988)).

The HREs are in fact structurally related but functionally distinct. The glucocorticoid receptor response element (GRE), estrogen receptor response element (ERE), and thyroid hormone receptor response element (TRE) have been characterized in detail. These particular response elements have been found to have a palindromic pair of hexameric "half-sites" (Evans, (1988); Green and Chambon, (1988)). With optimized pseudo- or consensus response elements, only two nucleotides per half-site differ between GRE and ERE (Klock et al., Nature 329:734-736 (1987)). On the other hand, EREs and TREs have identical half-sites but the number of nucleotide spacers between the two half sites is different (Glass et al., Cell 54:313-323 (1988)).

In contrast to response elements having the palindromic sequence motif, the following hormone receptors typically recognize response elements having two half-sites in a direct-repeat (DR) sequence motif: RXR, RAR, COUP-TF, PPAR, and the like (see, e.g., Mangelsdorf et al., The Retinoids: Biology. Chemistry, and Medicine, 2nd Edition, Raven Press, Ltd., New York, 1994, Chapter 8). Thus at least three distinct means are used to achieve HRE diversity: 1) binding site specificity for a particular half-site; 2) nucleotide spacing between the two half-sites; and 3) the orientation of the half-sites to one another.

In insect systems, a pulse of the steroid hormone ecdysone triggers metamorphosis in Drosophila melanogaster showing genomic effects, such as chromosomal puffing, within minutes of hormone addition. Mediating this response in insects is the functional ecdysone receptor, a heterodimer of the ecdysone receptor (EcR) and the product of the ultraspiracle gene (USP) (Yao et al. (1993) Nature 366, 476-479; and Yao et al. (1992) Cell 71, 63-72). Responsiveness to an insect ecdysteroid can be recreated in cultured mammalian cells by co-transfection of EcR, USP, an ecdysone responsive reporter, and treatment with ecdysone or the synthetic analog muristerone A.

In the field of genetic engineering, precise control of gene expression is an invaluable tool in studying, manipulating and controlling development and other physiological processes. For example applications for regulated gene expression in mammalian systems include inducible gene targeting, overexpression of toxic and teratogenic genes, anti-sense RNA expression, and gene therapy (Jaenisch, R. (1988) Science 240, 1468-1474). For cultured cells, glucocorticoids and other steroids have been used to induce the expression of a desired gene.

As another means for controlling gene expression in a mammalian system, an inducible tetracycline regulated system has been devised and utilized in transgenic mice, whereby gene activity is induced in the absence of the antibiotic and repressed in its presence (see, e.g, Gossen et al. (1992) Proc. Natl. Acad. Sci. 89, 5547-5551; Gossen et al.(1993) TIBS 18, 471-475; Furth et al. (1994) Proc. Natl. Acad. Sci. 91, 9302-9306; and Shockett et al. (1995) Proc. Natl. Acad. Sci. 92, 6522-6526). However, disadvantages of this system include the continuous treatment of tetracycline to repress expression and the slow clearance of antibiotic from bone which interferes with quick and precise induction. While this system has been improved by the recent identification of a mutant tetracycline repressor which acts conversely as an inducible activator, the pharmacokinetics of tetracycline may hinder its use during development when a precise and efficient "on-off" switch is essential (Gossen et al. (1995) Science 268, 1766-1769).

Accordingly, there is a need in the art for improved methods to precisely modulate the expression of exogenous genes in mammalian subjects.

### BRIEF DESCRIPTION OF THE INVENTION

In accordance with the present invention, there are provided various methods for modulating the expression of an exogenous gene in a mammalian subject. The invention method is useful in a wide variety of applications where inducible in vivo expression of an exogenous gene is desired, such as in vivo therapeutic methods for delivering recombinant proteins into a variety of cells within a patient.

Unlike prior art tetracycline based strategies, transferring ecdysone responsiveness to mammalian cells takes advantage of a naturally evolved steroid inducible system. Advantages of ecdysteroid use include the lipophilic nature of the compounds (which provides efficient penetrance thereof into all tissues, including the brain), short half-lives (which allow for precise and potent inductions), and favorable pharmacokinetics that prevent storage and expedite clearance.

In accordance with another embodiment of the present invention, there are provided modified ecdysone receptors, which can be in the form of homodimeric species or heterodimeric species comprising at least one silent partner of the steroid/thyroid superfamily of receptors, along with an invention modified ecdysone receptor. Invention modified ecdysone receptors are useful, for example, in methods for modulating expression of an exogenous gene in a mammalian subject.

In accordance with additional embodiments of the present invention, there are provided nucleic acids encoding invention modified ecdysone receptors, modified ecdysone receptor response elements, gene transfer vectors, recombinant cells, and transgenic animals containing nucleic acid encoding invention modified ecdysone receptor.

### BRIEF DESCRIPTION OF THE FIGURES

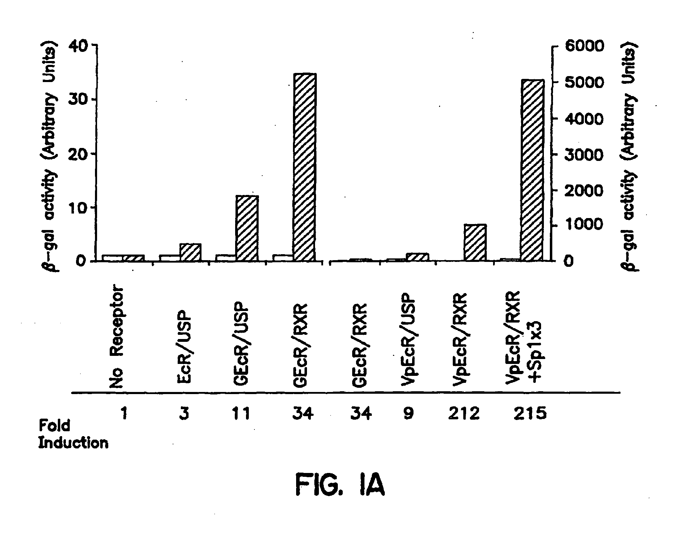

Figures 1A - 1D show the optimization of ecdysone responsiveness using various combinations of USP or RXR with different modified EcRs. In Figure 1A, the numerical values on both sides of the figure are on the same scale, with the GEcR/RXR value repeated for clarity. Darkened and stripped bars represent reporter activity with no hormone or 1µM muristerone A, respectively.

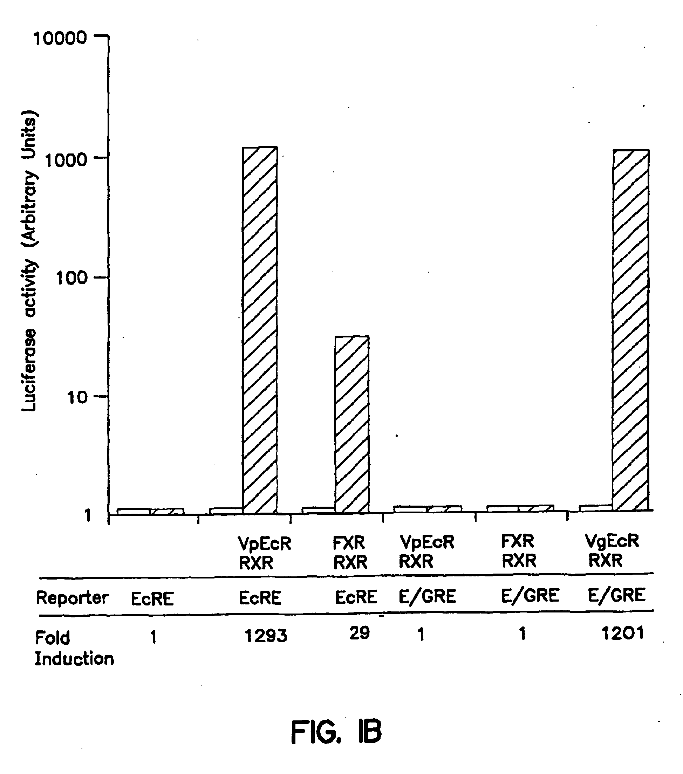

Figure 1B shows FXR and VpEcR activity on ecdysone response element (EcRE) and a hybrid ecdysone/glucocorticoid response element (E/GRE) responsive reporters. VpEcR, VgEcR, and control transfection without receptors were treated with 1µM muristerone. FXR transfections were treated with 50µM Juvenile Hormone III (Sigma). Darkened and stripped bars represent reporter activity with no hormone or 1µM muristerone A/50µM Juvenile Hormone III, respectively.

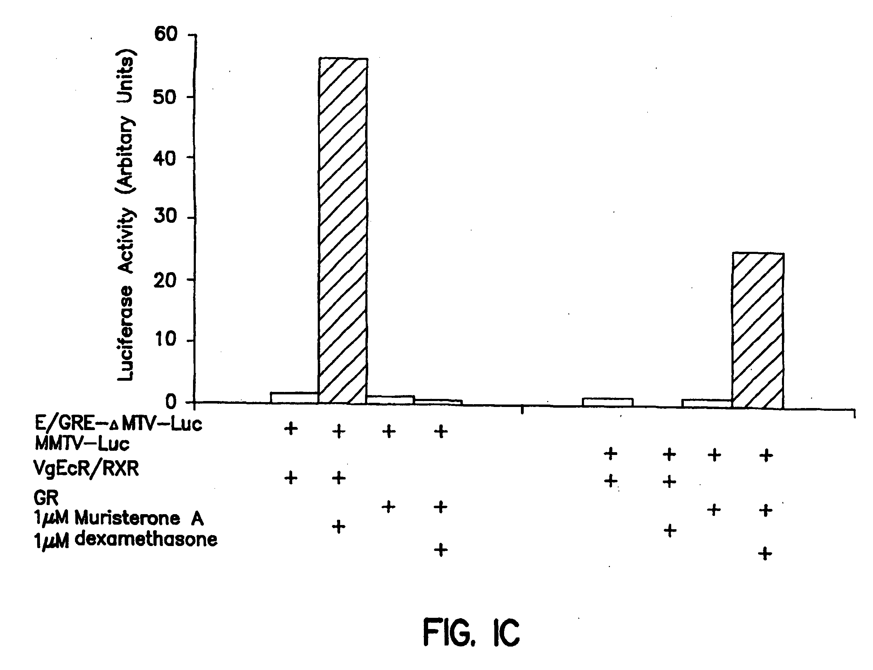

Figure 1C shows that E/GRE and GRE are non-overlapping response elements. Darkened and stripped bars represent reporter activity with no hormone or 1µM muristerone A/1µM dexamethasone, respectively.

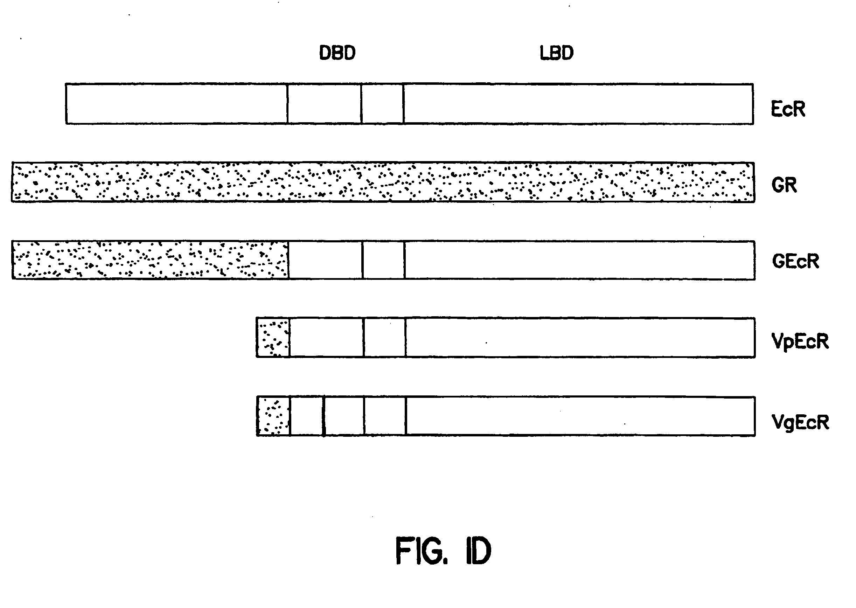

Figure 1D shows a schematic diagram of modified ecdysone receptors. GEcR is a chimeric receptor containing the N-terminal transactivation domain of GR and the DNA- and ligand-binding domains of EcR. VpEcR is an N-terminal truncation of EcR fused to the activation domain of Vp16. VgEcR is identical to VpEcR except for the following point mutations in the P box of the DNA binding domain: E282G, G283S, and G286V. In the Figure, DBD=DNA binding domain and LBD=ligand binding domain.

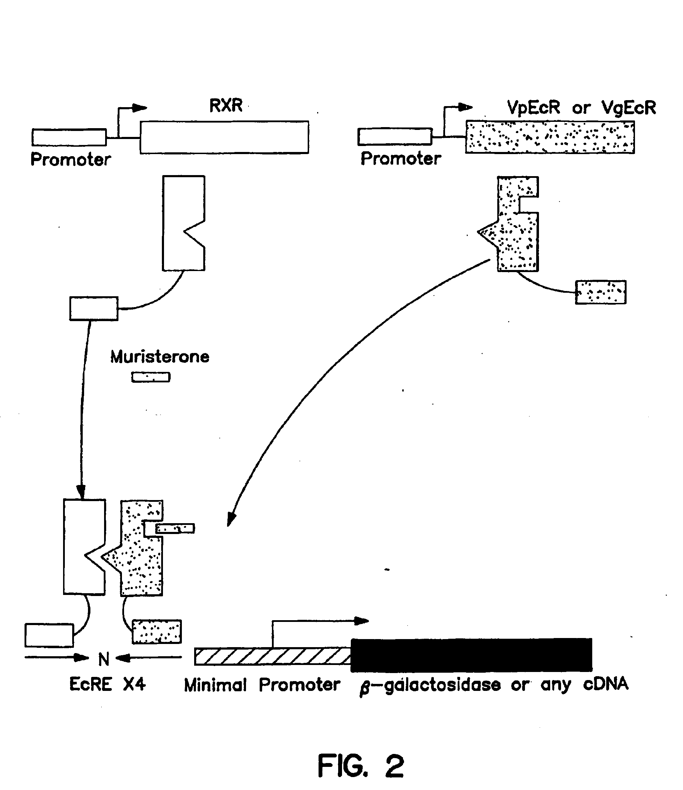

Figure 2 shows a schematic diagram of an invention ecdysone inducible gene expression system. After expression of RXR and a modified EcR, the two receptors can heterodimerize and transactivate the ecdysone response element-containing promoter in the presence of hormone. The ecdysone response elements are placed upstream of a minimal promoter (i.e., an enhancerless promoter) which can drive the expression of any exogenous cDNA.

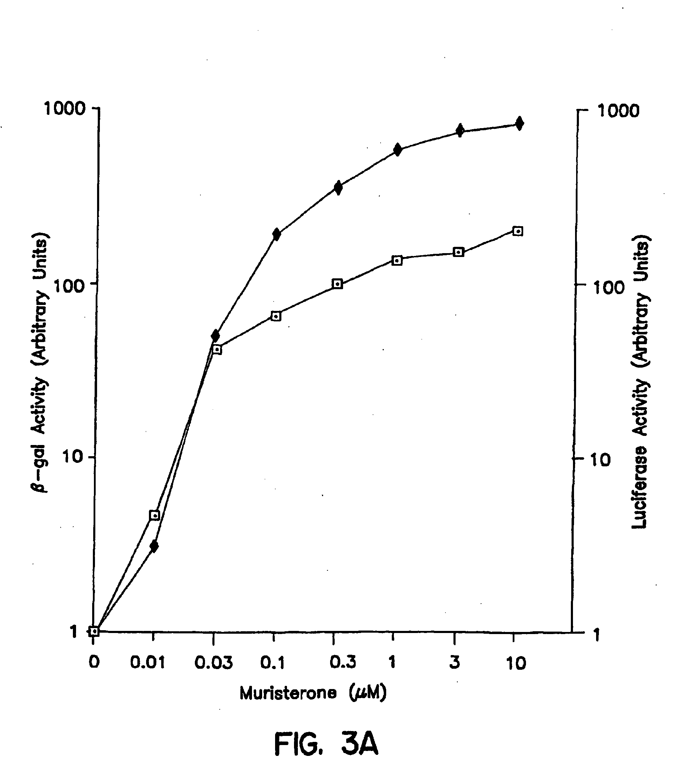

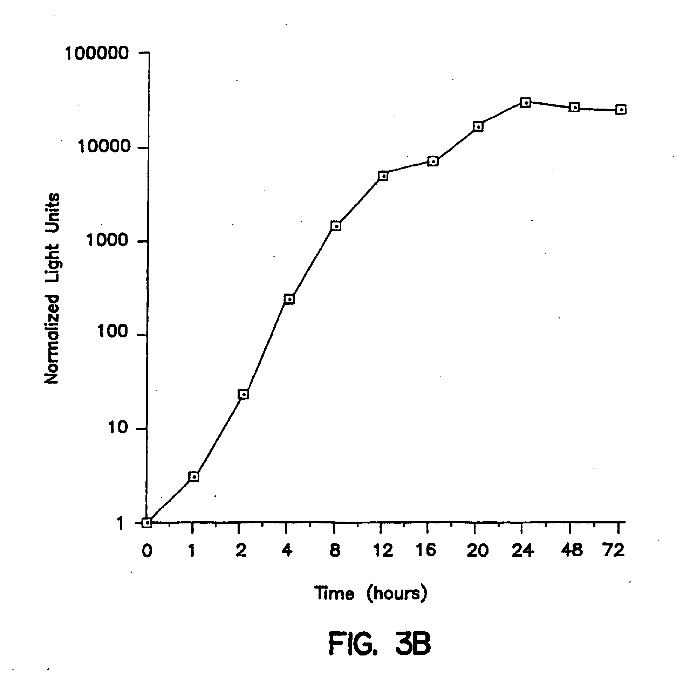

Figure 3A shows a dose-dependent activation of N13 cells with muristerone. N13 cells were grown with varying concentrations of muristerone for 36 hours and then assayed for β-galactosidase activity (open squares) by standard ONPG assay or for luciferase activity (closed circles). Figure 3B shows the time-course of luciferase activity of N13 cells treated with hormone. N13 cells were grown in separate wells in the presence of 1µM muristerone, harvested at varying times, and assayed for luciferase activity as described in Example 3.

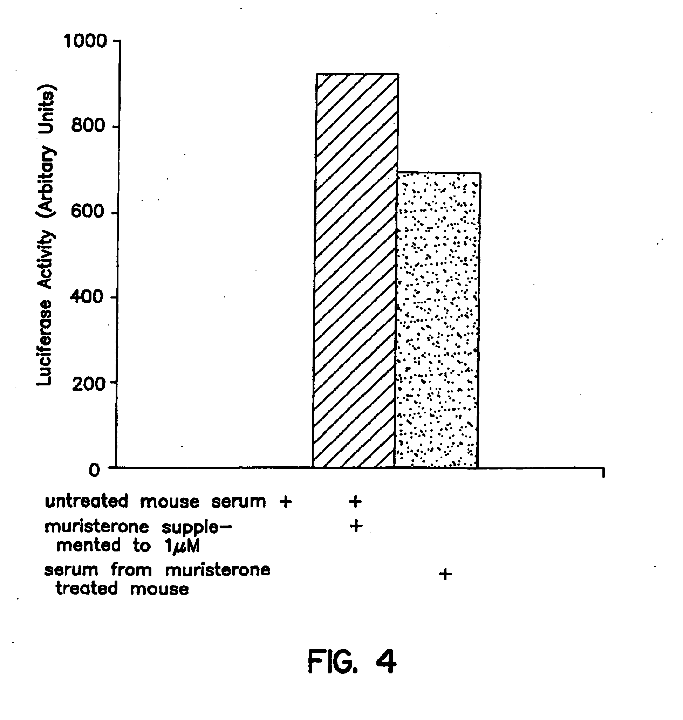

Figure 4 shows muristerone activity in mice as described in Example 4.

### DETAILED DESCRIPTION OF THE INVENTION

The present invention provides a method in accordance with claim 1.

In accordance with the present disclosure, there are further provided methods for modulating the expression of an exogenous gene in a mammalian subject containing:
(i) a DNA construct comprising said exogenous gene under the control of an ecdysone response element; and
(ii) a modified ecdysone receptor which, in the presence of a ligand therefor, and optionally in the further presence of a receptor capable of acting as a silent partner therefor, binds to said ecdysone response element;
said method comprising administering to said subject an effective amount of a ligand for said modified ecdysone receptor; wherein said ligand is not normally present in the cells of said subject; and wherein said ligand is not toxic to said subject.

Thus, in accordance with the present invention the insect molting hormone, ecdysone, is advantageously employed as a regulated inducer of gene expression in mammalian systems, i.e., background levels of expression are substantially zero in the absence of conditions required for induction. It has been found that optimized promoters containing a novel modified ecdysone response element in conjunction with an invention modified ecdysone receptor (preferably having an altered DNA binding specificity) provide an extremely powerful and specific inducible mammalian expression system. The low basal activity of the invention expression system is advantageously suitable for the expression of transcription factors and toxic genes. The excellent dose response and induction rate characteristics of the invention inducible expression system allow for precise control of both the degree and duration of induction of a desired gene.

Since the invention method provides for regulated gene expression by an exogenous non-mammalian inducer, it can be advantageously employed in a variety of in vivo and in vitro mammalian expression systems. For example, inducible expression of cre recombinase in transgenic mammals, in accordance with invention methods, would enable those of skill in the art to accomplish temporally specific inducible gene targeting of the adult or the developing embryo (O'Gorman et al. (1991) Science 251, 1351-1355).

As employed herein, the terms "modulate" and "modulating" refer to the ability of a given ligand/receptor complex to effect transactivation of transcription of an exogenous gene, relative to such ability of said receptor in the absence of ligand. The actual effect of complex formation on the transactivation activity of a receptor will vary depending on the specific receptor species which are part of the ligand/receptor complex, and on the response element with which the ligand/receptor complex interacts.

As used herein, when referring to genes, the phrase "exogenous to said mammalian subject" or simply "exogenous" refers to any gene wherein the gene product is not naturally expressed in the particular cell where expression is desired. For example, exogenous genes can be either natural or synthetic wild type genes and therapeutic genes, which are introduced into the subject in the form of DNA or RNA. The gene of interest can be introduced into target cells (for in vitro applications), or the gene of interest can be introduced directly into a subject, or indirectly introduced by the transfer of transformed cells into a subject.

"Wild type" genes are those that are native to cells of a particular type. Such genes may be undesirably overexpressed, or may not be expressed in biologically significant levels. Thus, for example, while a synthetic or natural gene coding for human insulin would be exogenous genetic material to a yeast cell (since yeast cells do not naturally contain insulin genes), a human insulin gene inserted into a human skin fibroblast cell would be a wild type gene with respect to that cell since human skin fibroblasts contain genetic material encoding human insulin, although human skin fibroblasts do not express human insulin in biologically significant levels.

Wild type genes contemplated for use in the practice of the present invention include genes which encode a gene product:
the substantial absence of which leads to the occurrence of a non-normal state in said subject; or
a substantial excess of which leads to the occurrence of a non-normal state in said subject;
and the like.

As employed herein, the phrase "therapeutic gene" refers to a gene which imparts a beneficial function to the host cell in which such gene is expressed. Therapeutic genes are those that are not naturally found in host cells. For example, a synthetic or natural gene coding for wild type human insulin would be therapeutic when inserted into a skin fibroblast cell so as to be expressed in a human host, where the human host is not otherwise capable of expressing functionally active human insulin in biologically significant levels. In accordance with the methods described herein, therapeutic genes are expressed at a level that provides a therapeutically effective amount of the corresponding therapeutic protein.

Therapeutic genes contemplated for use in the practice of the present invention include genes which encode a gene product:
which is toxic to the cells in which it is expressed; or
which imparts a beneficial property to the host subject (e.g., disease resistance, etc);
and the like.

Numerous genomic and cDNA nucleic acid sequences coding for a variety of proteins are well known in the art. Exogenous genetic material useful in the practice of the present invention include genes that encode biologically active proteins of interest, such as, e.g., secretory proteins that can be released from said cell; enzymes that can metabolize a substrate from a toxic substance to a nontoxic substance, or from an inactive substance to a useful substance; regulatory proteins; cell surface receptors; and the like. Useful genes include genes that encode blood clotting factors such as human factors VIII and IX; genes that encode hormones such as insulin, parathyroid hormone, luteinizing hormone releasing factor (LHRH), alpha and beta seminal inhibins, and human growth hormone; genes that encode proteins such as enzymes, the absence of which leads to the occurrence of an abnormal state; genes encoding cytokines or lymphokines such as interferons, granulocytic macrophage colony stimulating factor (GM-CSF), colony stimulating factor-1 (CSF-1), tumor necrosis factor (TNF), and erythropoietin (EPO); genes encoding inhibitor substances such as alpha, -antitrypsin; genes encoding substances that function as drugs, e.g., genes encoding the diphtheria and cholera toxins; and the like.

Typically, nucleic acid sequence information for a desired protein can be located in one of many public access databases, e.g., GENBANK, EMBL, Swiss-Prot, and PIR, or in many biology related journal publications. Thus, those of skill in the art have access to nucleic acid sequence information for virtually all known genes. Those of skill in the art can either obtain the corresponding nucleic acid molecule directly from a public depository or the institution that published the sequence. Optionally, once the nucleic acid sequence encoding a desired protein has been ascertained, the skilled artisan can employ routine methods, e.g., polymerase chain reaction (PCR) amplification, to isolate the desired nucleic acid molecule from the appropriate nucleic acid library. Thus, all known nucleic acids encoding proteins of interest are available for use in the methods and products described herein.

As used herein, the terms "mammal" and "mammalian" refer to humans; domesticated animals, e.g., rats, mice, rabbits, canines, felines, and the like; farm animals, e.g., chickens, bovine, porcine and ovine, and the like; and animals of zoological interest, e.g., monkeys and baboons, and the like.

Modified ecdysone receptors contemplated for use in the present disclosure comprise:
an ecdysone ligand binding domain;
a DNA-binding domain obtained from a DNA-binding protein; and
an activation domain of a transcription factor,
wherein at least one of said DNA-binding domain or said activation domain is not obtained from a native ecdysone receptor,
with the proviso that when said activation domain is derived from a glucocorticoid receptor, said DNA-binding domain is not derived from a glucocorticoid receptor or an E. coli LexA protein. In accordance with the present disclosure, modified ecdysone receptors function in expression systems, preferably mammalian, to transactivate gene expression from transcription regulatory regions having ecdysone response elements.

Ecdysone ligand binding domains contemplated for use in the preparation of modified ecdysone receptors of the present disclosure are typically derived from the carboxy-terminal portion of native ecdysone receptor and are able to bind ecdysteroids (Koelle et al., Cell, 67:59-77, 1991; and Christopherson et al., PNAS. USA, 89:6314-6318, 1992). Ecdysone ligand binding domains can be functionally located in either orientation and at various positions within the modified ecdysone receptor of the invention. For example, the ecdysone ligand binding domain can be positioned at either the amino or carboxy terminus of the modified receptor, or therebetween. Preferably, the ecdysone ligand binding domain is positioned at the carboxy terminus of the modified receptor (see Figure 1D).

DNA-binding domains contemplated for use in the preparation of modified ecdysone receptors of the present disclosure are typically obtained from DNA-binding proteins (e.g., transcription factors). The term "DNA-binding domain" is understood in the art to refer to an amino acid sequence that is able to bind to DNA. As used herein, the term "DNA-binding domain" encompasses a minimal peptide sequence of a DNA-binding protein, up to the entire length of a DNA-binding protein, so long as the DNA-binding domain functions to associate with a particular response element.

Such DNA-binding domains are known to function heterologously in combination with other functional protein domains by maintaining the ability to bind the natural DNA recognition sequence (see, e.g., Brent and Ptashne, 1985, Cell, 43:729-736). For example, hormone receptors are known to have interchangeable DNA-binding domains that function in chimeric proteins (see, e.g., U.S. Patent 4,981,784; and Evans, R., 1988, Science, 240:889-895). Thus, similar to the ligand binding domain of modified ecdysone receptor of the present disclosure, the DNA-binding domain can be positioned at either the carboxy terminus or the amino terminus, or the DNA-binding domain can be positioned between the ligand binding domain and the activation domain. Preferably, the DNA-binding domain is positioned internally between the ligand binding domain and the activation domain.

"DNA-binding protein(s)" contemplated for use herein belong to the well-known class of proteins that are able to directly bind DNA and facilitate initiation or repression of transcription. Exemplary DNA-binding proteins contemplated for use herein include transcription control proteins (e.g., transcription factors and the like; Conaway and Conaway, 1994, "Transcription Mechanisms and Regulation", Raven Press Series on Molecular and Cellular Biology, Vol. 3, Raven Press, Ltd., New York, NY).

Transcription factors contemplated for use herein as a source of such DNA binding domains include, e.g., homeobox proteins, zinc finger proteins, hormone receptors, helix-turn-helix proteins, helix-loop-helix proteins, basic-Zip proteins (bzip), β-ribbon factors, and the like. See, for example, Harrison, s., "A Structural Taxonomy of DNA-binding Domains," Nature, 353:715-719. Homeobox DNA-binding proteins suitable for use herein include, for example, HOX, STF-1 (Leonard et al., 1993, Mol. Endo., 7:1275-1283), Antp, Mat α-2, INV, and the like. See, also, Scott et al. (1989), Biochem. Biophys. Acta, 989:25-48. It has been found that a fragment of 76 amino acids (corresponding to amino acids 140-215 described in Leonard et al., 1993, Mol. Endo., 7:1275-1283) containing the STF-1 homeodomain binds DNA as tightly as wild-type STF-1. Suitable zinc finger DNA-binding proteins for use herein include Zif268, GLI, XFin, and the like. See also, Klug and Rhodes (1987), Trends Biochem. Sci., 12:464; Jacobs and Michaels (1990), New Biol.,2:583; and Jacobs (1992), EMBO J., 11:4507-4517.

Preferably, the DNA-binding domain used herein is obtained from a member of the steroid/thyroid superfamily of receptors. As used herein, the phrase "member(s) of the steroid/thyroid hormone superfamily of receptors" (also known as "nuclear receptors" or "intracellular receptors") refers to hormone binding proteins that operate as ligand-dependent transcription factors, including identified members of the steroid/thyroid superfamily of receptors for which specific ligands have not yet been identified (referred to hereinafter as "orphan receptors").

Exemplary members of the steroid/thyroid superfamily of receptors (including the various isoforms thereof) include steroid receptors such as glucocorticoid receptor (GR), mineralocorticoid receptor (MR), estrogen receptor (ER), progesterone receptor (PR), androgen receptor (AR), vitamin D₃ receptor (VDR), and the like; plus retinoid receptors, such as the various isoforms of retinoic acid receptor (e.g., RARα, RARβ, or RARγ), the various isoforms of retinoid X receptor (e.g., RXRα, RXRβ, or RXRγ), and the like (see, e.g., U.S. Patents 4,981,784; 5,171,671; and 5,071,773); thyroid receptors (TR), such as TRα, TRβ, and the like; insect derived receptors such as the ecdysone receptor, and the like; as well as other gene products which, by their structure and properties, are considered to be members of the superfamily, as defined hereinabove, including the various isoforms thereof. Examples of orphan receptors contemplated for use herein as a source of DNA binding domain include HNF4 [see, for example, Sladek et al., in Genes & Development 4: 2353-2365 (1990)], the COUP family of receptors (see, for example, Miyajima et al., in Nucleic Acids Research 16: 11057-11074 (1988), and Wang et al., in Nature 340: 163-166 (1989)], COUP-like receptors and COUP homologs, such as those described by Mlodzik et al., in Cell 60: 211-224 (1990) and Ladias et al., in Science 251: 561-565 (1991), various isoforms of peroxisome proliferator-activated receptors (PPARs; see, for example, Issemann and Green, supra), the insect derived knirps and knirps-related receptors, and the like.

The DNA-binding domains of all members of the steroid/thyroid superfamily of receptors are related, consisting of 66-68 amino acid residues, and possessing about 20 invariant amino acid residues, including nine cysteines. A member of the superfamily can be characterized as a protein which contains these 20 invariant amino acid residues. The highly conserved amino acids of the DNA-binding domain of members of the superfamily are as follows: wherein X designates non-conserved amino acids within the DNA-binding domain; an asterisk denotes the amino acid residues which are almost universally conserved, but for which variations have been found in some identified hormone receptors; and the residues enclosed in parenthesis are optional residues (thus, the DNA-binding domain is a minimum of 66 amino acids in length, but can contain several additional residues).

Modification of existing DNA-binding domains to recognize new target recognition sequences is also contemplated herein. For example, it has been found that the modification of the "P-box" sequence of DNA-binding domains of members of the steroid/thyroid superfamily of receptors offers unique advantages not present in other chimeric hormone receptors. For example, the modification of a P-box amino acid sequence to preferentially bind to a different hormone response element half-site than the naturally occurring P-box amino acid sequence can reduce undesired background levels of gene expression. Thus, disclosed receptors and methods provide the advantage of increasing the selectivity of exogenous gene expression in a particular subject.

As used herein, the phrase "P-box amino acid sequence" refers to the proximal element region in a DNA-binding domain of a hormone receptor that typically occurs at the junction of the first zinc finger and the linker region, e.g., at about amino acids 19-23 of the DNA-binding domain (i.e., amino acids 19-23 of SEQ ID NO:1; see, e.g., Umesono et al. (1989), Cell. 57:1139-1146, Figure 2). Umesono et al. (1989), supra, in Table 1, describe various naturally occurring P-box amino acid sequences for a variety of hormone receptor DNA-binding domains.

According to the present disclosure, the P-box sequence of a hormone receptor DNA-binding domain is modified to have a P-box amino acid sequence that differs from the naturally occurring P-box amino acid sequence. Preferably, the modified P-box amino acid sequence differs from the naturally occurring P-box amino acid sequence by 3 amino acids.

Preferably, the P-box amino acid sequence is modified so that only the half-site nucleotide sequence recognized by the DNA-binding domain is changed while not altering the spacing between the two half-sites recognized by the DNA-binding domain. For example, when the DNA-binding domain of the ecdysone receptor is employed in a modified ecdysone receptor of the present disclosure, the P-box can be modified from the amino acid sequence EGCKG (SEQ ID NO:2; which recognizes the half-site -AGGTCA-) to the amino acid sequence GSCKV (SEQ ID NO:3; which recognizes the half-site seqeunce -AGAACA-). Preferably, when the DNA-binding domain of modified ecdysone receptor of the present disclosure is derived from ecdysone receptor, the P-box amino acid sequence is modified to GSCKV (SEQ ID NO:3).

It has also been found that in vitro evolution methods can be applied to modify and improve existing DNA-binding domains (see, e.g., Devlin et al., 1990, Science, 249:404-406; and Scott and Smith, 1990, Science. 249:386-390).

Activation domains contemplated for use in the preparation of modified ecdysone receptor are typically derived from transcription factors and comprise a contiguous sequence of amino acids that functions to activate gene expression when associated with a suitable DNA-binding domain and a suitable ligand binding domain. As with the ligand and DNA-binding domains employed for the preparation of modified ecdysone receptors, the activation domain can be positioned at the carboxy terminus, the amino terminus or between the ligand binding domain and the DNA binding domain. Preferably, the activation domain is positioned at the amino terminus of the modified ecdysone receptor.

Suitable activation domains can be obtained from a variety of sources, e.g., from the N-terminal region of a member of the steroid/thyroid superfamily of receptors, from a transcription factor activation domain, such as, for example, VP16 or GAL4 activation domains, and the like. The presently most preferred activation domain contemplated for use is obtained from the N-terminal region of the VP16 protein.

The presently most preferred modified ecdysone receptors contemplated for use herein are VgEcR (SEQ ID NO:5), VpEcR (SEQ ID NO:7) or GEcR (SEQ ID NO:9), with VgEcR (SEQ ID NO:5) being especially preferred. The preparation of these modified ecdysone receptors is set forth hereinafter in Example 1.

Modified ecdysone receptor proteins of the present disclosure can be produced by expressing nucleic acid constructs encoding the chimeric proteins in suitable host cells as described in Example 1. Recombinant methods of producing desired proteins by introducing an expression construct into appropriate host cells are well-known in the art. Modified ecdysone receptors of the present disclosure can be introduced into a particular subject by direct introduction of the proteins themselves, by introducing DNA construct(s) encoding the receptor into the subject, or into cells obtained from the subject (wherein the cells are transformed and subsequently returned to the subject).

Preferably, modified ecdysone receptors are expressed under the control of a tissue specific promoter. As readily understood by those of skill in the art, the term "tissue specific" refers to the substantially exclusive initiation of transcription in the tissue from which a particular promoter drives expression of a given gene.

In accordance with one aspect of the present disclosure, modified ecdysone receptors are present in the form of heterodimeric species comprising an modified ecdysone receptor and at least one silent partner of the steroid/thyroid superfamily of receptors. Preferably, the silent partner is a mammalian-derived receptor, with RXR being especially preferred.

Silent partners contemplated herein are members of the steroid/thyroid superfamily of receptors which are capable of forming heterodimeric species with the modified ecdysone receptor of the present disclosure, wherein the silent partner does not directly participate in binding ligand (i.e., only the modified ecdysone receptor co-partner of the heterodimer binds ligand). The silent partner can either be endogenous to the cells of the subject or can be provided to the subject by introducing DNA construct(s) encoding receptor into the subject. A preferred silent partner for use herein is RXR. In a particular aspect of the disclosed methods, exogenous RXR is provided to said mammalian subject.

The formation of heterodimeric receptor(s) can modulate the ability of member(s) of the steroid/thyroid superfamily of receptors to trans-activate transcription of genes maintained under expression control in the presence of ligand for said receptor. For example, formation of a heterodimer of the modified ecdysone receptor with another mammalian hormone receptor promotes the ability of the modified ecdysone receptor to induce trans-activation activity in the presence of an ecdysone response element.

In accordance with another aspect of the present disclosure, modified ecdysone receptors are present in the form of homodimeric species comprising a plurality (i.e., at least two) disclosed modified ecdysone receptors.

Ligands contemplated for use herein are compounds which, inside a cell, bind to disclosed modified ecdysone receptors, thereby creating a ligand/receptor complex, which in turn can bind to an appropriate response element. The terms "ecdysone" and "ecdysteroid" as interchangeably used herein, are employed herein in the generic sense (in accordance with common usage in the art), referring to a family of ligands with the appropriate binding and transactivation activity (see, for example, Cherbas et al., in Biosynthesis. metabolism and mode of action of invertebrate hormones (ed. J. Hoffmann and M. Porchet), p. 305-322; Springer-Verlag, Berlin). An ecdysone, therefore, is a compound which acts to modulate gene transcription for a gene maintained under the control of an ecdysone response element.

20-Hydroxy-ecdysone (also known as β-ecdysone) is the major naturally occurring ecdysone. Unsubstituted ecdysone (also known as α-ecdysone) is converted in peripheral tissues to β-ecdysone. Analogs of the naturally occurring ecdysones are also contemplated within the scope of the present invention. Examples of such analogs, commonly referred to as ecdysteroids, include ponasterone A, 26-iodoponasterone A, muristerone A, inokosterohe, 26-mesylinokosterone, and the like. Since it has been previously reported that the above-described ecdysones are neither toxic, teratogenic, nor known to affect mammalian physiology, they are ideal candidates for use as inducers in cultured cells and transgenic mammals according to the invention methods.

Ligands contemplated for use in the practice of the present invention are characterized as not normally being present in the cells of the subject, meaning that the ligand is exogenous to the subject. Ecdysteroids, for example, are not naturally present in mammalian systems. Thus, in accordance with the invention method, unless and until an ecdysteroid is administered to the subject, substantially no expression of the desired gene occurs.

An effective amount of ligand contemplated for use in the practice of the present invention is the amount of ligand (i.e., ecdysteroid) required to achieve the desired level of gene expression product. Ligand can be administered in a variety of ways, as are well-known in the art. For example, such ligands can be administered topically, orally, intravenously, intraperitoneally, intravascularly, and the like.

Ecdysone response elements contemplated for use in the present disclosure (relating to modulation of the expression of exogenous genes in a subject) include native, as well as modified ecdysone response elements. Since modified ecdysone receptors of the present disclosure can function as either homodimers or as heterodimers (with a silent partner therefor), any response element that is responsive to a modified ecdysone receptor of the present disclosure, in the form of a homodimer or heterodimer, is contemplated for use in the methods described herein.

Preferably according to the present disclosure, modified ecdysone response elements are engineered so as to no longer be capable of binding to a farnesoid hormone receptor (since the mammalian farnesoid hormone receptor is able to bind to native ecdysone receptor response element). Disclosed modified ecdysone response elements provide low background expression levels of the exogenous gene and increase the selectivity of the gene expression system when used in mammalian systems.

Ecdysone response elements contemplated for use herein are short cis-acting sequences (i.e., having about 12-20 bp) that are required for activation of transcription in response to a suitable ligand, such as ecdysone or muristerone A, associated with a particular hormone receptor. The association of these response elements with otherwise ecdysone-nonresponsive regulatory sequences causes such regulatory sequences to become ecdysone responsive. Ecdysone response element sequences function in a position- and orientation-independent fashion.

The native ecdysone response element has been previously described, see, e.g., Yao et al., Cell, 71:63-72, 1992. Modified ecdysone response elements according to present disclosure comprise two half-sites (in either direct repeat or inverted repeat orientation to one another), separated by a spacer of 0-5 nucleotides. As used herein, the term "half-site" refers to a contiguous 6 nucleotide sequence that is bound by a particular member of the steroid/thyroid superfamily of receptors. Each half-site is typically separated by a spacer of 0 up to about 5 nucleotides. Typically, two half-sites with a corresponding spacer make up a hormone response element. Hormone response elements can be incorporated in multiple copies into various transcription regulatory regions.

Preferred modified ecdysone response elements according to the disclosure comprise, in any order, a first half-site and a second half-site separated by a spacer of 0-5 nucleotides;
wherein the first and second half-sites are inverted with respect to each other;
wherein said first half-site has the sequence:
-RGBNNM-,
(or complements thereof) wherein
each R is independently selected from A or G; each B is independently selected from G, C, or T; each N is independently selected from A, T, C, or G; and
each M is independently selected from A or C;
with the proviso that at least 4 nucleotides of each -RGBNNM- group of nucleotides are identical with the nucleotides at comparable positions of the sequence -AGGTCA-; and
said second half-site is obtained from a glucocorticoid receptor subfamily response element.

The complement to the -RGBNNM- sequence set forth above is:
-YCVNNK-,
wherein
each Y is independently selected from T or C:
each V is independently selected from C, G, or A;
each N is independently selected from A, T, C, or G; and
each K is independently selected from T or G.

Exemplary first half-sites having the -RGBNNM- motif for use in the invention modified ecdysone response element include, for example, half-sites selected from -AGGGCA-, -AGTTCA-, -AGGTAA-, -AGGTCA-, -GGTTCA-, -GGGTTA-, -GGGTGA-, -AGGTGA-, or -GGGTCA-. A particularly preferred first half-site is -AGTGCA-.

Glucocorticoid receptor subfamily response elements contemplated for use in the present disclosure are response elements having half-sites that are typically bound by glucocorticoid, mineralocorticoid, progesterone or androgen receptors. Suitable half-sites from glucocorticoid receptor subfamily response elements can be selected from the following sequence (in either orientation):
-RGNNCA-
(or complements thereof such as -YCNNGT-), wherein R, Y and N are as defined above. Exemplary half-sites having the -RGNNCA- motif for use in the invention modified ecdysone response element include -AGAACA-, -GGAACA-, -AGTTCA-, -AGGTCA-, -GGAACA-, -GGTTCA-, -GGGTCA-, -GGGTCA-, -AGGTGA-, -GGGTCA-, and the like, as well as complements thereof. Particularly preferred half-sites having the -RGNNCA- motif include -AGAACA- and -GGAACA-, with -AGAACA- being especially preferred.

When the above-described modified ecdysone response elements are employed to bind heterodimeric receptors of the present disclosure, the second half-site is inverted with respect to the first half-site. For example, when describing a single-strand of an invention modified ecdysone response element in the 5'-3' direction, the following general motif can be employed:
RGBNNM-(N)ₓ-TGNNCY (SEQ ID NO:10),
where x is an integer of 0 up to about 5, with x = 1 being especially preferred. As an alternative orientation to the above described response element motif (SEQ ID NO:10), an invention response element can be described in the 5'-3' direction as:
RGNNCA-(N)ₓ-KNNVCY (SEQ ID NO:11), where x is an integer of 0 up to about 5, with x = 1 being especially preferred.

In preferred aspects of the present disclosure, the first half-site is obtained from an ecdysone response element and the second half-site is obtained from a hormone response element selected from a glucocorticoid response element, a mineralocorticoid response element, a progesterone response element or an androgen response element. In a particularly preferred aspect of the present disclosure, the first half-site is obtained from an ecdysone response element and the second half-site is obtained from a glucocorticoid response element.

In a particularly preferred aspect of the disclosure modified ecdysone response element, the first half-site is AGTGCA and said second half-site is TGTTCT. The presently most preferred modified-ecdysone response element for use in the invention methods is:
AGTGCA-N-TGTTCT (SEQ ID NO:12).

In another aspect of the disclosure, when modified ecdysone receptors of the disclosure exist as homodimers, response elements employed preferably have a direct repeat motif (instead of the above-described inverted repeat motif), as follows:
RGBNNM-(N)ₓ,-RGBNNM (SEQ ID NO:13),
where R, B, N and M are as previously defined, and x' is an integer of 0 up to about 5, with x' = 3 being especially preferred.

Disclosed modified ecdysone response elements are characterized as having substantially no constitutive activity, which refers to the substantial absence of background levels of gene expression initiated by invention modified ecdysone response elements when introduced into mammalian expression systems. Since it has been found that mammalian farnesoid hormone receptors are able to bind to and transactivate gene expression from native ecdysone response elements, in certain aspects of the present disclosure (e.g., where it is desired to avoid farnesoid-mediated background expression), modified ecdysone response elements are employed.

Presently preferred modified ecdysone response elements are further characterized as having substantially no binding affinity for farnesoid X receptor (FXR), i.e., response elements are incapable of binding FXR (which would thereby create undesired background levels of expression). Thus, presently preferred modified ecdysone response elements preferably induce basal levels of expression of substantially zero.

Response elements employed in the practice of the present disclosure are operably linked to a suitable promoter for expression of exogenous gene product(s). As used herein, the term "promoter" refers to a specific nucleotide sequence recognized by RNA polymerase, the enzyme that initiates RNA synthesis. This sequence is the site at which transcription can be specifically initiated under proper conditions. When exogenous genes, operatively linked to a suitable promoter, are introduced into the cells of a suitable host, expression of the exogenous genes is controlled by the presence of ecdysteroid compounds, which are not normally present in the host cells.

In accordance with another aspect of the present disclosure, there are provided methods of inducing the expression of an exogenous gene in a mammalian subject containing:
(i) a DNA construct comprising an exogenous gene under the control of an ecdysone response element,
(ii) DNA encoding a modified ecdysone receptor under the control of an inducible promoter; wherein said modified ecdysone receptor, in the presence of a ligand therefor, and optionally in the further presence of a receptor capable of acting as a silent partner therefor, binds to said ecdysone response element, and
(iii) a ligand for said modified ecdysone receptor;
said method comprising subjecting said subject to conditions suitable to induce expression of said modified ecdysone receptor.

Inducible promoters contemplated for use in the practice of the present disclosure are transcription regulatory regions that do not function to transcribe mRNA unless inducing conditions are present. Examples of suitable inducible promoters include DNA sequences corresponding to: the E. coli lac operator responsive to IPTG (see Nakamura et al., Cell, 18:1109-1117, 1979); the metallothionein promoter metal-regulatory-elements responsive to heavy-metal (e.g. zinc) induction (see Evans et. al, U.S. Patent No. 4,870,009), the phage T71ac promoter responsive to IPTG (see Studier et al., Meth. Enzymol., 185: 60-89, 1990; and U.S. #4,952,496), the heat-shock promoter, and the like.

In accordance with another aspect of the present disclosure, there are provided methods of inducing expression of an exogenous gene in a mammalian subject containing a DNA construct comprising said exogenous gene under the control of an ecdysone response element, said method comprising introducing into said subject:
a modified ecdysone receptor; and
a ligand for said modified ecdysone receptor,
wherein said receptor, in combination with a ligand therefor, and optionally in the further presence of a receptor capable of acting as a silent partner therefor, binds to said ecdysone response element, activating transcription therefrom.

In accordance with another aspect of the present disclosure, there are provided methods for the expression of recombinant products detrimental to a host organism, said method comprising:
transforming suitable host cells with:
   (i) a DNA construct encoding said recombinant product under the control of an ecdysone response element, and
   (ii) DNA encoding a modified ecdysone receptor;
growing said host cells in suitable media; and
inducing expression of said recombinant product by introducing into said host cells ligand(s) for said modified ecdysone receptor, and optionally a receptor capable of acting as a silent partner for said modified ecdysone receptor.

Recombinant products detrimental to a host organism contemplated for expression in accordance with the present invention include any gene product that functions to confer a toxic effect on the organism. For example, inducible expression of a toxin such as the diptheroid toxin would allow for inducible tissue specific ablation (Ross et al. (1993) Genes and Development 7, 1318-1324). Thus, the numerous gene products that are known to induce apoptosis in cells expressing such products are contemplated for use herein (see, e.g, Apoptosis, The Molecular Basis of Cell Death, Current Communications In Cell & Molecular Biology, Cold Spring Harbor Laboratory Press, 1991).

Suitable media contemplated for use in the practice of the present invention include any growth and/or maintenance media, in the substantial absence of ligand(s) which, in combination with an invention modified ecdysone receptor, is(are) capable of binding to an ecdysone response element.

In accordance with another embodiment of the present invention, there are provided nucleic acids encoding invention modified ecdysone receptors. Invention nucleic acids can be incorporated into various expression vectors known to those of skill in the art. Preferred nucleic acids encoding modified ecdysone receptors are set forth in SEQ ID NOs:4, 6 and 8, with SEQ ID NO:4 being especially preferred.

In accordance with another embodiment of the present invention, there are provided gene transfer vectors useful for the introduction of invention constructs into suitable host cells. Such gene transfer vectors comprise a transcription regulatory region having a minimal promoter (i.e., a promoter region that does not have an enhancer), and an invention modified ecdysone response element, wherein said regulatory region is operatively associated with DNA containing an exogenous gene, and wherein said modified ecdysone response element is present in multiple copies. The number of copies of response elements can readily be varied by those of skill in the art. For example, transcription regulatory regions can contain from 1 up to about 50 copies of a particular response element, preferably 2 up to about 25 copies, more preferably 3 up to about 10-15 copies, with about 4-6 copies being especially preferred.

Gene transfer vectors (also referred to as "expression vectors") contemplated for use herein are recombinant nucleic acid molecules that are used to transport exogenous nucleic acid into cells for expression and/or replication thereof. Expression vectors may be either circular or linear, and are capable of incorporating a variety of nucleic acid constructs therein. Expression vectors typically come in the form of a plasmid that, upon introduction into an appropriate host cell, results in expression of the inserted DNA.

As used herein, the phrase "transcription regulatory region" refers to the region of a gene or expression construct that controls the initiation of mRNA transcription. Regulatory regions contemplated for use herein typically comprise at least a minimal promoter in combination with an ecdysone response element. A minimal promoter, when combined with an enhancer region (e.g., a hormone response element), functions to initiate mRNA transcription in response to a ligand/receptor complex. However, transcription will not occur unless the required inducer (ligand) is present.

As used herein, the phrase "operatively associated with" refers to the functional relationship of DNA with regulatory and effector sequences of nucleotides, such as promoters, enhancers, transcriptional and translational stop sites, and other signal sequences. For example, operative linkage of DNA to a promoter refers to the physical and functional relationship between the DNA and promoter such that the transcription of such DNA is initiated from the promoter by an RNA polymerase that specifically recognizes, binds to and transcribes the DNA.

Preferably, the transcription regulatory region further comprises a binding site for an ubiquitous transcription factor. Such a binding site is preferably positioned between the promoter and modified ecdysone response element of the invention. Suitable ubiquitous transcription factors for use herein are well-known in the art and include, for example, Sp1.

Expression vectors suitable for use in the practice of the present invention are well known to those of skill in the art and include those that are replicable in eukaryotic cells and/or prokaryotic cells as well as those that remain episomal and those that integrate into the host cell genome. Expression vectors typically further contain other functionally important nucleic acid sequences, such as expression constructs encoding antibiotic resistance proteins, and the like.

Exemplary eukaryotic expression vectors include eukaryotic constructs, such as the pSV-2 gpt system (Mulligan et al., Nature, 1979, 277:108-114); pBlueSkript (Stratagene, La Jolla, CA), the expression cloning vector described by Genetics Institute (Science, 1985, 228:810-815), and the like. Each of these plasmid vectors are capable of promoting expression of the invention chimeric protein of interest.

Promoters, depending upon the nature of the regulation, may be constitutively or inducibly regulated, or may be tissue-specific (e.g., expressed only in T-cells, endothelial cels, smooth muscle cells, and the like). Exemplary promoters contemplated for use in the practice of the present invention include the SV40 early promoter, the cytomegalovirus (CMV) promoter, the mouse mammary tumor virus (MMTV) steroid-inducible promoter, Moloney murine leukemia virus (MMLV) promoter, elongation factor 1α (EF1α) promoter, albumin promoter, APO A1 promoter, cyclic AMP dependent kinase II (CaMKII) promoter, keratin promoter, CD3 promoter, immunoglobulin light or heavy chain promoters, neurofiliment promoter, neuron specific enolase promoter, L7 promoter, CD2 promoter, myosin light chain kinase promoter, HOX gene promoter, thymidine kinase (TK) promoter, RNA Pol II promoter, MYOD promoter, MYF5 promoter, phophoglycerokinase (PGK) promoter, Stfl promoter, Low Density Lipoprotein (LDL) promoter,and the like.

Suitable means for introducing (transducing) expression vectors containing invention nucleic acid constructs into host cells to produce transduced recombinant cells (i.e., cells containing recombinant heterologous nucleic acid) are well-known in the art (see, for review, Friedmann, 1989, Science, 244:1275-1281; Mulligan, 1993, Science, 260:926-932, each of which are incorporated herein by reference in their entirety). Exemplary methods of transduction include, e.g., infection employing viral vectors (see, e.g., U.S. Patent 4,405,712 and 4,650,764), calcium phosphate transfection (U.S. Patents 4,399,216 and 4,634,665), dextran sulfate transfection, electroporation, lipofection (see, e.g., U.S. Patents 4,394,448 and 4,619,794), cytofection, particle bead bombardment, and the like. The heterologous nucleic acid can optionally include sequences which allow for its extrachromosomal (i.e., episomal) maintenance, or the heterologous nucleic acid can be donor nucleic acid that integrates into the genome of the host.

In a specific embodiment, said gene transfer vector is a viral vector, preferably a retroviral vector. Retroviral vectors are gene transfer plasmids that have an expression construct encoding an heterologous gene residing between two retroviral LTRs. Retroviral vectors typically contain appropriate packaging signals that enable the retroviral vector, or RNA transcribed using the retroviral vector as a template, to be packaged into a viral virion in an appropriate packaging cell line (see, e.g., U.S. Patent 4,650,764).

Suitable retroviral vectors for use herein are described, for example, in U.S. Patents 5,399,346 and 5,252,479; and in WIPO publications WO 92/07573, WO 90/06997, WO 89/05345, WO 92/05266 and WO 92/14829, incorporated herein by reference, which provide a description of methods for efficiently introducing nucleic acids into human cells using such retroviral vectors. Other retroviral vectors include, for example, mouse mammary tumor virus vectors (e.g., Shackleford et al., 1988, PNAS, USA, 85:9655-9659), and the like.

Various procedures are also well-known in the art for providing helper cells which produce retroviral vector particles which are essentially free of replicating virus. See, for example, U.S. Patent 4,650,764; Miller, Human Gene Therapy, 1:5-14 (1990); Markowitz, et al., Journal of Virology, 61(4):1120-1124 (1988) ; Watanabe, et al., Molecular and Cellular Biology, 3(12):2241-2249 (1983); Danos, et al., Proc. Natl. Acad. Sci., 85:6460-6464 (1988); and Bosselman, et al., Molecular and Cellular Biology, 7(5):1797-1806 (1987), which disclose procedures for producing viral vectors and helper cells which minimize the chances for producing a viral vector which includes a replicating virus.

Recombinant retroviruses suitable for carrying out the invention methods are produced employing well-known methods for producing retroviral virions. See, for example, U.S. Patent 4,650,764; Miller, Human Gene_Therapy, 1:5-14 (1990); Markowitz, et al., Journal of Virology, 61(4):1120-1124 (1988); Watanabe, et al., Molecular and Cellular Biology, 3(12):2241-2249 (1983); Danos, et al., Proc. Natl. Acad. Sci., 85:6460-6464 (1988); and Bosselman, et al., Molecular and Cellular Biology, 7(5):1797-1806 (1987).

In accordance with another embodiment of the present invention, there are provided recombinant cells containing a nucleic acid encoding an invention modified ecdysone receptor. Exemplary eukaryotic cells for introducing invention expression vectors include, e.g., CV-1 cells, P19 cells and NT2/D1 cells (which are derived from human embryo carcinomas), COS cells, mouse L cells, Chinese hamster ovary (CHO) cells, primary human fibroblast cells, human embryonic kidney cells, African green monkey cells, HEK 293 (ATCC accession #CRL 1573; U.S. Patent No. 5,024,939), Ltk⁻ cells (ATCC accession #CCL1.3), COS-7 cells (ATCC under accession #CRL 1651), DG44 cells (dhfr⁻ CHO cells; see, e.g., Urlaub et al. (1986) Cell. Molec. Genet. 12: 555), cultured primary tissues, cultured tumor cells, and the like. Presently preferred cells include CV-1 and 293 cells.

In accordance with another embodiment of the present invention, there is provided a transgenic mammal containing a nucleic acid encoding an inveniton modified ecdysone receptor. As used herein, the phrase "transgenic mammal" refers to a mammal that contains one or more inheritable expression constructs containing a recombinant modified ecdysone receptor transgene and/or an exogenous gene under the transcription control of an invention modified ecdysone response element. Preferably, an invention transgenic mammal also contains one or more inheritable expression constructs containing a member of the steroid/thyroid superfamily of receptors that functions as a silent partner for modified ecdysone receptor (e.g., RXR).

Methods of making transgenic mammals using a particular nucleic acid construct are well-known in the art. When preparing invention transgenic animals, it is preferred that two transgenic lines are generated. The first line will express, for example, RXR and a modified EcR (e.g., VpEcR). Tissue specificity is conferred by the selection of tissue-specific promoters (e.g., T-cell specific) that will then direct the expression of the receptors. A second line contains an ecdysone responsive promoter controlling the expression of an exogenous cDNA.

In a preferred embodiment of the present invention, an invention transgenic mammal contains one or more expression constructs containing nucleic acid encoding a modified ecdysone receptor, exogenous RXR, and an exogenous gene under the transcription control of an invention modified ecdysone response element. It has been found that in transgenic mice containing an ecdysone inducible promoter (i.e., an invention modified ecdysone response element) and expressing a modified ecdysone receptor and RXR, muristerone treatment can activate gene expression. Thus, with tissue specific expression of the modified ecdysone receptor and RXR and timely hormone treatment, inducible gene expression can be achieved with spatial, dosage, and temporal specificity.

In accordance with another aspect of the present disclosure, there are provided methods for inducing expression of an exogenous gene in a transgenic mammal containing a modified ecdysone receptor according to the disclosure, said method comprising:
introducing into said mammal a DNA construct encoding an exogenous gene under the transcription control of an ecdysone response element responsive to said modified ecdysone receptor; and
administering to said mammal an amount of ligand for said modified ecdysone receptor effective to induce expression of said exogenous gene.

As discussed hereinbefore, the modified ecdysone receptor forms a homodimer, or optionally a heterodimer in the presence of a silent partner of the steroid/thyroid hormone superfamily of receptors, and functions to activate transcription from an expression vector having a response element responsive to the particular homodimer or heterodimer formed.

In accordance with another aspect of the present disclosure, there are provided methods for the induction of two different genes in a mammalian subject comprising: activating a first exogenous gene employing the invention ecdysone inducible system; and activating a second gene using a tetracycline inducible system. The invention method for inducing two different genes is particularly advantagous because it permits the temporal, spatial, and dosage specific control of two exogenous genes.

The tetracycline inducible system is well-known in the art (see, e.g, Gossen et al. (1992) Proc. Natl. Acad. Sci. 89, 5547-5551; Gossen et al.(1993) TIBS 18, 471-475; Furth et al. (1994) Proc. Natl. Acad. Sci. 91, 9302-9306; and Shockett et al. (1995) Proc. Natl. Acad. Sci. 92, 6522-6526).

All U.S. and Foreign Patent publications, textbooks, and journal publications referred to herein are hereby expressly incorporated by reference in their entirety. The invention will now be described in greater detail by reference to the following non-limiting examples.

### Example 1

### Preparation of modified ecdysone receptors

### Plasmid preparation:

The plasmids CMX-EcR, CMX-USP, CMX-FXR, CMX-hRXRa, EcREx5-ΔMTV-Luc, CMX-GEcR, MMTV-luc, and CMX-GR have been previously described (Yao, et al., Nature 366:476-479 (1993) and Forman, et al. Cell 81:687-693 (1995)).

The plasmid CMX-VpEcR was constructed by ligation of an EcoRI fragment of psk-EcR and CMX-Vp16.

The plasmid CMX-VgEcR was generated by site-directed mutagenesis of CMX-VpEcR using the Transformer Mutagenesis Kit (Clontech) and the mutagenic Oligonucleotide (SEQ ID NO:14):
5'-TACAACGCCCTCACCTGTGGATCCTGCAAGGTGTTTCTTTCGACGCAGC-3'.

Mutagenesis of VpEcR to VgEcR altered the P-box region of the DNA binding domain of ecdysone receptor to resemble that of GR (Umesono and Evans, Cell 57:1139-1146 (1989)). The following amino acids in the DNA-binding domain of the ecdysone receptor were altered: E282G, G283S, and G286V (E=glutamate, G=glycine, S=serine, V=valine).

The reporter construct EcREx4-ΔHSP-ß-gal was constructed by oligomerizing two annealed oligonucleotides containing the HSP-EcRE (Yao, et al., Nature 366:476-479 (1993)).

EcREx4-Sp1x3-ΔHSP-ßgal was constructed by ligating the following annealed oligonucleotides into the Asp718 site of EcREx4-HSP-ß-gal (SEQ ID NO:15):
5'-GTACTCCCGGGGCGGGGCTATGCGGGGCGGGGCTAATCGCTAGGGGCGGGGCA-3'
   and (SEQ ID NO:16):
5'-GTACTGCCCCGCCCCTAGCGATTAGCCCCGCCCCGCATAGCCCCGCCC CGGGA-3'.
ΔHSP is a minimal promoter derived from the Drosophila heat shock promoter with its enhancers deleted.

To generate the construct E/GREx4-ΔMTV-Luc, the following oligonucleotides (SEQ ID NO:17):
5'-AGCTCGATGGACAAGTGCATTGTTCTTTGCTGAA-3';
   and (SEQ ID NO:18):
5'-AGCTTTCAGCAAGAGAACAATGCACTTGTCCATCG-3',
   were annealed, multimerized, and ligated into the HindIII site of ΔMTV-Luc. The resulting reporter contained 4 copies of the invention modified ecdysone response element E/GRE.

To produce the plasmid pRC-ESHB, a BglII/(XhoI) fragment containing EcREx4-Sp1x3-ΔHSP-ß-gal was subcloned into BglII/(NotI) digested pRC-CMV (Invitrogen, San Diego, CA), which contains a neomycin resistance gene.

### Cell Culture and Transient Transfections:

CV-1 cells were maintained in DMEM supplemented with 10% Fetal Bovine Serum. Transient transfections were performed using DOTAP transfection reagent (Boehringer-Mannheim). Transfections using ß-galactosidase as the reporter were assayed either by Galactolight luminescent assay (Tropix, Bedford, MA) or by standard liquid ONPG assay (Sigma, St. Louis, MO). The values were normalized by co-transfection of CMX-luciferase. Transfections using luciferase as the reporter were assayed by standard techniques using luciferin and ATP. These values were normalized by co-transfection of CMX-ß-galactosidase. Hormone treated cells were treated with ethanol, 50 µM Juvenile Hormone III (Sigma), 1µM muristerone A (Zambon, Bresso, IT), or 1µM dexamethasone (Sigma) unless otherwise noted.

To maximize the sensitivity of the invention ecdysone inducible system, modifications of the ecdysone receptor were made. The N-terminal transactivation domain of the ecdysone receptor was replaced by the corresponding domain of the glucocorticoid receptor (GR), resulting in the modified ecdysone receptor GEcR (See Figure 1D). CV-1 cells were transfected with the plasmid CMX-GEcR encoding the modified ecdysone receptor as discussed above. After transfection, cells were either treated with ethanol or 1µM muristerone A. This hybrid modified ecdysone receptor boosted muristerone responsiveness from 3- to 11-fold in a transient transfection assay (Fig. 1A). Replacement of the natural heterodimeric partner for the ecdysone receptor, USP, by its mammalian homologue, the retinoid X receptor (RXR), produced a more potent ligand dependent heterodimer, providing a 34 fold induction (Fig. 1A).

A more potent heterodimer, however, was obained by combining RXR and VpEcR, an N-terminal truncation of the ecdysone receptor attached to the VP16 activation domain, resulting in a 212 fold induction (Fig. 1A and 1D). Different from most nuclear receptor/VP16 fusion proteins which exhibit high constitutive activity, VpEcR generates ligand dependent superinduction while maintaining a very low basal activity (Underhill et al., Mol. Encod. 8:274-285 (1994) and Perlmann et al., Genes & Devel. 7:1411-1422 (1993)).

In addition, the reporter vector was also modified by inserting consensus binding sites for the ubiquitous transcription factor Sp1 between the minimal promoter and the ecdysone response elements (Kamine et al., Proc. Natl. Acad. Sci. 88:8510-8514 (1991) and Strahle te al., EMBO 7:3389-3395 (1988)). The addition of Sp1 sites to the ecdysone responsive promoter increases the absolute activity 5-fold (Fig. 1A).

### Example 2

### Construction of a novel ecdysone response element

Although no mammalian transcription factors have been shown to have a natural enhancer element like the ecdysone response element, which is composed of two inverted half-sites of the sequence AGGTCA spaced by one nucleotide, it is difficult to preclude such a possibility. The recently cloned farnesoid X receptor (FXR) can very weakly activate certain synthetic promoters containing an ecdysone response element in response to extremely high concentrations of farnesoids (Forman et al., Cell 81:687-693 (1995)).

In FXR containing cells and in transgenic mice, activation of gene expression by endogenous receptors would create undesirable background levels of reporter protein. To circumvent this potential problem, the DNA binding specificity of VpEcR was altered to mimic that of GR, which binds as a homodimer to an inverted repeat of the sequence AGAACA, spaced by three nucleotides. This altered binding specificity was achieved by mutating three amino acid residues of VpEcR in the P-box of the DNA binding domain, a region previously shown to be essential for DNA sequence recognition (Umesono and Evans, Cell 57:1139-1146 (1989)). This new hybrid modified ecdysone receptor is referred herein as VgEcR and is responsive to a new hybrid respone element referred to herein as the E/GRE (SEQ ID NO:12), which contains two different half-site motifs, RGBNNM and RGNNCA, spaced by one nucleotide (Fig. 1B). This new response element is a hybrid between the glucocorticoid response element (GRE) and that of type II nuclear receptors like RXR, EcR, retinoic acid receptor (RAR), thyroid hormone receptor (T3R), etc. Although FXR can activate a promoter containing the wild type ecdysone response element, it cannot activate one containing the E/GRE (Fig 1B; note log scale). The E/GRE reporter is not activated by GR nor does VgEcR activate a dexamethasone responsive promoter (Fig 1C).

### Example 3

### Assay for Ecdysone responsiveness in stable cell lines

Stable cell lines were generated containing the modified ecdysone receptor VpEcR, a heterodimeric partner (RXR), and an ecdysone inducible reporter (Figure 2). 293 cells were transfected with the following linearized plasmids, pRC-ESHß, EcREx5-ΔMTV-Luc, CMX-VpEcR, and CMX-hRXRa. The following day, the cells were split 1:10 and were allowed to recover one day prior to selection with 1mg/ml G418 (GIBCO). After 14 days of selection, 14 individual clones were isolated and grown separately in the presence of 0.5mg/ml G418. Of 14 G418 resistant clones, 10 demonstrated differing degrees of muristerone responsiveness.

One of these cell lines, N13, was grown in the presence or absence of 1µM muristerone for 20 hours. Cell lysates were then assayed for ß-galactosidase and luciferase activities as described in Example 1. X-gal staining was performed on the stable cell lines. Cells were fixed briefly with 10% formaldehyde in PBS and then stained with X-Gal (Molecular Probes, Eugene, OR) for 2 to 6 hours. After 24 hours of treatment with 1µM muristerone, 100% of the cells turned dark blue after 3 hours of staining. Thus, mammalian cells containing the modified ecdysone receptor VpEcR, a heterodimeric partner (RXR), and a reporter gene construct regulated by a modified ecdysone response element, function to efficiently express an exogenous gene in response to a ligand, e.g., ecdysone.

A dose-response assay was conducted by growing N13 cells with varying concentrations of muristerone for 36 hours and then assaying for ß-galactosidase activity (using the well-known ONPG assay), or the cells were assayed for luciferase activity. Dose response curves of stably integrated ß-galactosidase and luciferase reporters in N13 cells revealed that inducibility approaching 3 orders of magnitude can be achieved at a final concentration 10µM muristerone (Figure 3A). One-hundred fold induction was achieved by muristerone concentrations as low as 100nM (Figure 3A).

Finally, the kinetics of muristerone mediated induction was measured. N13 cells were grown in separate wells in the presence of 1µM muristerone, harvested at varying times, and assayed for luciferase activity. Inductions of 100-fold in 3 hrs., 1000 fold in 8 hrs., and maximal effects of 20,000 fold after 20 hours of treatment were observed (Figure 3B). Similar results were observed in stable lines containing CMX-VgEcR and the E/GRE reporters.

### Example 4

### Bioavailability and activity of muristerone

In order to use muristerone as a potential hormone in mice, its toxicity and bioavailability was examined. For toxicity studies, adult mice were injected intraperitonealy with 20mg of muristerone A suspended in sesame oil. The mice were then observed for approximately two months. For teratogenic studies, pregnant mice were injected with 20mg of muristerone A suspended in sesame oil and both the mother and pups were observed for three months. The results indicate that muristerone maintains its activity when injected into mice, and that it is neither toxic, teratogenic, nor inactivated by serum binding proteins. In addition to the inert qualities of muristerone (an ecdysone), overexpression of VpEcR and RXR appears not to be toxic.

For muristerone bioavailability studies, adult mice were injected intraperitoneally with sesame oil with or without 10mg of muristerone, and were subsequently sacrificed for serum collection. After twelve hours, blood was drawn from the mice, and the serum was isolated by brief centrifugation of the whole blood. In order to conduct transfection assays to test for muristerone activity, serum from sesame oil injected mice was divided, and half was supplemented with muristerone to a final concentration of 10µM. The three batches of mouse serum were diluted 1:10 in DMEM and placed onto CV-1 cells transfected with CMX-GEcR, CMX-hRXRa, and EcREx5-DMTV-Luc.

The results are shown in Figure 4 and indicate that serum from muristerone treated mice yielded a luciferase activity comparable to that seen from untreated mouse serum supplemented with 1µM muristerone. The results indicate that single-site injected material should be widely circulated, and that there is little or no blunting of activity due to association with serum proteins.

### Example 5

### Muristerone dependent gene expression in transgenic mice

To produce transgenic mice, the following DNA constructs were prepared and subsequently injected into fertilized eggs: CD3-VpEcR, CD3-RXR, ESHß (Lee et al., J. Exp. Med. 175:1013-1025 (1992)). Two separate lines of transgenic mice were generated harboring either an ecdysone inducible reporter, ESHß, or a T-cell specific expression construct of VpEcR and RXR, respectively. The former are referred to as reporter mice, the latter are referred to as receptor mice, and double transgenic mice are referred to as receptor/reporter mice. Constructs CD3-VpEcR and CD3-RXR were mixed and coinjected, while ESHß was injected alone. Primary genotyping was performed by Southern blot analysis and the transmission of transgenic mice was monitored by dot blot analysis. Receptor mice were analyzed for VpEcR and RXR expression by Northern blot analysis of RNA collected from these mice. For Northern blot analysis, 15µg of total RNA obtained from the thymus, and various tissues as a control, was run on a denaturing gel and blotted onto a nitrocellulose membrane. The blot was probed with a radiolabeled ß-gal-specific probe and exposed on film for 2 days. These receptor mice were healthy, fertile, and appeared normal by visual inspection. In addition, the transgene was transferred to the offspring as expected by Mendelian genetics. This data suggests that overexpression of VpEcR and RXR in T-cells is not toxic.

Receptor expressing mice were bred with reporter mice (containing ESHß) to produce double transgenic receptor/reporter mice. Adult receptor/reporter transgenic mice (genotype=CD3-VpEcR; CD3-RXR: and ESHβ) were injected intraperitonealy with sesame oil with or without 10mg of muristerone. Subsequently, a Northern blot analysis was performed on the double transgenic lines using RNA isolated 48 hours after treatment from various tissues including the thymus, brain and liver, to test for the specific induction of an ecdysone inducible promoter. The probe used was specific to the activity of the ecdysone inducible promoter. The autoradiograph was exposed for 36 hrs. The results of the Northern analysis indicate that muristerone treatment of the transgenic mouse containing a T-cell specific expression construct of VpEcR and RXR, and the ecdysone inducible reporter ESHß, caused a significant induction from an ecdysone inducible promoter in the thymus, while low basal activity is observed in its absence.

## Claims

1. An in vitro method for modulating the expression of an exogenous gene in an isolated cell containing:
(i) the modified ecdysone receptor VgEcR having the amino acid sequence set forth in SEQ ID NO:5; and
(ii) a DNA construct comprising said exogenous gene under the control of a modified ecdysone response element, wherein said modified response element is E/GRE having the nucleotide sequence set forth in SEQ ID NO:12;
said method comprising providing to the cell an effective amount of one or more ecdysteroids.

2. A recombinant cell comprising:
(i) the modified ecdysone receptor VgEcR having the amino acid sequence set forth in SEQ ID NO:5; and
(ii) a DNA construct comprising said exogenous gene under the control of a modified ecdysone response element, wherein said modified response element is E/GRE having the nucleotide sequence set forth in SEQ ID NO:12.

3. An expression system for modulating the expression of an exogenous gene in an isolated cell comprising,
(i) the modified ecdysone receptor VgEcR having the amino acid sequence set forth in SEQ ID NO:5; and
(ii) a DNA construct comprising said exogenous gene under the control of a modified ecdysone response element, wherein said modified response element is E/GRE having the nucleotide sequence set forth in SEQ ID NO:12.

4. A modified ecdysone receptor VgEcR having the amino acid sequence set forth in SEQ ID NO:5.

5. A nucleic acid encoding the modified ecdysone receptor according to claim 4.

6. A modified ecdysone receptor response element E/GRE having the nucleotide sequence set forth in SEQ ID NO:12.

7. A gene transfer vector comprising a transcription regulatory region having a minimal promoter, and a modified ecdysone response element according to claim 6, wherein said regulatory region is operatively associated with DNA containing an exogenous gene, and wherein said modified ecdysone response element is present in 1 up to about 6 copies.

8. A vector according to claim 7, wherein said regulatory region further comprises a binding site for a ubiquitous transcription factor.

9. A vector according to claim 8, wherein said binding site is positioned between said promoter and said synthetic ecdysone response element.

10. A vector according to claim 9, wherein said ubiquitous transcription factor is Spl.

11. A vector according to claim 7, wherein said promoter is tissue-specific.

12. An in vitro method for the expression of a recombinant product detrimental to a host organism, said method comprising:
transforming suitable host cells with:
(i) a DNA construct encoding said recombinant product under the control of the modified ecdysone response element E/GRE having the nucleotide sequence set forth in SEQ ID NO:12, and
(ii) DNA encoding the modified ecdysone receptor VgEcR having the amino acid sequence set forth in SEQ ID NO:5;
growing said host cells in suitable media; and
inducing expression of said recombinant product by introducing into said host cells an ecdysteroid.

13. Use of an effective amount of a ligand for the modified ecdysone receptor VgEcR having the amino acid sequence set forth in SEQ ID NO:5, wherein said ligand is an ecdysteroid, for the manufacture of a medicament for modulating the expression of an exogenous gene in a mammalian subject containing:
(i) a DNA construct comprising said exogenous gene under the control of the modified ecdysone response element E/GRE having the nucleotide sequence set forth in SEQ ID NO:12; and
(ii) the modified ecdysone receptor VgEcR having the amino acid sequence set forth in SEQ ID NO:5.

## Patentansprüche

1. In vitro-Verfahren zur Modulation der Expression eines exogenen Gens in einer isolierten Zelle, umfassend:
(i) den modifizierten Ecdyson-Rezeptor VgEcR mit der Aminosäuresequenz, die in SEQ ID NO: 5 dargestellt ist; und
(ii) ein DNA-Konstrukt, umfassend das genannte exogene Gen unter der Kontrolle eines modifizierten Ecdyson-Response-Elements, wobei das genannte modifizierte Response-Element E/GRE mit der Nukleotidsequenz ist, die in SEQ ID NO: 12 dargestellt ist;
wobei das genannte Verfahren Bereitstellen einer wirksamen Menge eines oder mehrerer Ecdysteroide für die Zelle umfasst.

2. Rekombinante Zelle, umfassend:
(i) den modifizierten Ecdyson-Rezeptor VgEcR mit der Aminosäuresequenz, die in SEQ ID NO: 5 dargestellt ist; und
(ii) ein DNA-Konstrukt, umfassend das genannte exogene Gen unter der Kontrolle eines modifizierten Ecdyson-Response-Elements, wobei das genannte modifizierte Response-Element E/GRE mit der Nukleotidsequenz ist, die in SEQ ID NO: 12 dargestellt ist.

3. Expressionssystem zur Modulation der Expression eines exogenen Gens in einer isolierten Zelle, umfassend
(i) den modifizierten Ecdyson-Rezeptor VgEcR mit der Aminosäuresequenz, die in SEQ ID NO: 5 dargestellt ist; und
(ii) ein DNA-Konstrukt, umfassend das genannte exogene Gen unter der Kontrolle eines modifizierten Ecdyson-Response-Elements, wobei das genannte modifizierte Response-Element E/GRE mit der Nukleotidsequenz ist, die in SEQ ID NO: 12 dargestellt ist;

4. Modifizierter Ecdyson-Rezeptor VgEcR mit der Aminosäuresequenz, die in SEQ ID NO: 5 dargestellt ist.

5. Nukleinsäure, die den Ecdyson-Rezeptor gemäß Anspruch 4 kodiert.

6. Modifiziertes Ecdyson-Rezeptor-Response-Element E/GRE mit der Nukleotidsequenz, die in SEQ ID NO: 12 dargestellt ist.

7. Gentransfervektor, umfassend einen Transkriptionsregulierenden Bereich mit einem minimalen Promotor und ein modifiziertes Ecdyson-Response-Element gemäß Anspruch 6, wobei der genannte regulatorische Bereich operativ mit DNA, die ein exogenes Gen enthält, verbunden ist und wobei das genannte modifizierte Ecdyson-Response-Element in 1 bis zu etwa 6 Kopien vorliegt.

8. Vektor gemäß Anspruch 7, wobei der genannte regulatorische Bereich ferner eine Bindungsstelle für einen ubiquitären Transkriptionsfaktor enthält.

9. Vektor gemäß Anspruch 8, wobei die genannte Bindungsstelle zwischen dem genannten Promotor und dem genannten synthetischen Ecdyson-Response-Element positioniert ist.

10. Vektor gemäß Anspruch 9, wobei der genannte ubiquitäre Transkriptionsfaktor Sp1 ist.

11. Vektor gemäß Anspruch 7, wobei der genannte Promotor gewebespezifisch ist.

12. In vitro-Verfahren zur Expression eines rekombinanten Produkts, das für den Wirtsorganismus schädlich ist, wobei das genannte Verfahren umfasst:
Transformieren geeigneter Wirtszellen mit:
(i) einem DNA-Konstrukt, das das genannte rekombinante Produkt unter der Kontrolle des modifizierten Ecdyson-Response-Elements E/GRE mit der Nukleotidsequenz, die in SEQ ID NO: 12 dargestellt ist; kodiert und
(ii) DNA, die den modifizierten Ecdyson-Rezeptor VgEcR mit der Aminosäuresequenz, die in SEQ ID NO: 5 dargestellt ist, kodiert;
Züchten der genannten Wirtszellen in geeigneten Medien; und
Induzieren der Expression des genannten rekombinanten Produkts durch Einführen eines Ecdysteroids in die genannten Wirtszellen.

13. Verwendung einer wirksamen Menge eines Liganden für den modifizierten Ecdyson-Rezeptor VgEcR mit der Aminosäuresequenz, die in SEQ ID NO: 5 dargestellt ist, wobei der genannte Ligand ein Ecdysteroid ist, zur Herstellung eines Medikaments zur Modulation der Expression eines exogenen Gens bei einem Säugetier, umfassend:
(i) ein DNA-Konstrukt, umfassend das genannte exogene Gen unter der Kontrolle des modifizierten Ecdyson-Response-Elements E/GRE mit der Nukleotidsequenz, die in SEQ ID NO: 12 dargestellt ist; und
(ii) den modifizierten Ecdyson-Rezeptor VgEcR mit der Aminosäuresequenz, die in SEQ ID NO: 5 dargestellt ist.

## Revendications

1. Procédé in vitro destiné à la modulation de l'expression d'un gène exogène dans une cellule isolée contenant :
(i) le récepteur de l'ecdysone modifié VgEcR ayant la séquence d'acides aminés présentée dans la SEQ ID NO : 5 ; et
(ii) une construction d'ADN comprenant ledit gène exogène sous le contrôle d'un élément de réponse à l'ecdysone modifié, où ledit élément de réponse modifié est l'élément E/GRE ayant la séquence nucléotidique présentée dans la SEQ ID NO : 12 ;
ledit procédé comprenant le fait de fournir à la cellule une quantité efficace d'un ou de plusieurs ecdystéroïde(s).

2. Cellule recombinante comprenant :
(i) le récepteur de l'ecdysone modifié VgEcR ayant la séquence d'acides aminés présentée dans la SEQ ID NO : 5 ; et
(ii) une construction d'ADN comprenant ledit gène exogène sous le contrôle d'un élément de réponse à l'ecdysone modifié, où ledit élément de réponse modifié est l'élément E/GRE ayant la séquence nucléotidique présentée dans la SEQ ID NO : 12.

3. Système d'expression destiné à la modulation de l'expression d'un gène exogène dans une cellule isolée comprenant,
(i) le récepteur de l'ecdysone modifié VgEcR ayant la séquence d'acides aminés présentée dans la SEQ ID NO : 5 ; et
(ii) une construction d'ADN comprenant ledit gène exogène sous le contrôle d'un élément de réponse à l'ecdysone modifié, où ledit élément de réponse modifié est l'élément E/GRE ayant la séquence nucléotidique présentée dans la SEQ ID NO : 12.

4. Récepteur de l'ecdysone modifié VgEcR ayant la séquence d'acides aminés présentée dans la SEQ ID NO : 5.

5. Acide nucléique codant pour le récepteur de l'ecdysone modifié selon la revendication 4.

6. Elément de réponse au récepteur de l'ecdysone modifié E/GRE ayant la séquence nucléotidique présentée dans la SEQ ID NO : 12.

7. Vecteur de transfert de gènes comprenant une région de régulation de la transcription ayant un promoteur minimal, et un élément de réponse à l'ecdysone modifié selon la revendication 6, où ladite région de régulation est associée de manière fonctionnelle à l'ADN contenant un gène exogène, et où ledit élément de réponse à l'ecdysone modifié est présent en 1 jusqu'à environ 6 copie(s).

8. Vecteur selon la revendication 7, dans lequel ladite région de régulation comprend en outre un site de liaison pour un facteur de transcription ubiquitaire.

9. Vecteur selon la revendication 8, dans lequel ledit site de liaison est positionné entre ledit promoteur et ledit élément de réponse à l'ecdysone synthétique.

10. Vecteur selon la revendication 9, dans lequel ledit facteur de transcription ubiquitaire est Spl.

11. Vecteur selon la revendication 7, dans lequel ledit promoteur est spécifique d'un tissu.

12. Procédé in vitro destiné à l'expression d'un produit recombinant nuisible à un organisme hôte, ledit procédé comprenant le fait :
de transformer des cellules hôtes appropriées avec :
(i) une construction d'ADN codant pour ledit produit recombinant sous le contrôle de l'élément de réponse à l'ecdysone modifié E/GRE ayant la séquence nucléotidique présentée dans la SEQ ID NO : 12, et
(ii) un ADN codant pour le récepteur de l'ecdysone modifié VgEcR ayant la séquence d'acides aminés présentée dans la SEQ ID NO : 5 ;
de mettre en culture lesdites cellules hôtes dans des milieux appropriés ; et
d'induire l'expression dudit produit recombinant par introduction d'un ecdystéroïde dans lesdites cellules hôtes.

13. Utilisation d'une quantité efficace d'un ligand pour le récepteur de l'ecdysone modifié VgEcR ayant la séquence d'acides aminés présentée dans la SEQ ID NO : 5, où ledit ligand est un ecdystéroïde, pour la fabrication d'un médicament destiné à la modulation de l'expression d'un gène exogène chez un sujet mammifère contenant :
(i) une construction d'ADN comprenant ledit gène exogène sous le contrôle de l'élément de réponse à l'ecdysone modifié E/GRE ayant la séquence nucléotidique présentée dans la SEQ ID NO : 12 ; et
(ii) le récepteur de l'ecdysone modifié VgEcR ayant la séquence d'acides aminés présentée dans la SEQ ID NO : 5.
